# EUROPÄISCHE PATENTSCHRIFT

(11) **EP 3 502 686 B1**
(45) Veröffentlichungstag und Bekanntmachung des Hinweises auf die Patenterteilung: **22.11.2023**
(21) Anmeldenummer: 18209802.0
(22) Anmeldetag: 03.12.2018
(51) Int. Cl.: G01N 33/12, G01N 33/53, G01N 33/569

(54) **ANTIGENNACHWEIS VON TRICHINELLA**
ANTIGEN DETECTION OF TRICHINELLA
DÉTERMINATION DE L'ANTIGÈNE DE TRICHINELLA

(30) Priorität: 20.12.2017 EP 17208994; 09.10.2018 EP 18199404
(43) Veröffentlichungstag der Anmeldung: 26.06.2019
(73) Patentinhaber: Euroimmun Medizinische Labordiagnostika AG, 23560 Lübeck (DE)
(72) Erfinder: Braasch, Jana, 23564 Lübeck (DE); Ostermann, Stefanie, 23847 Rethwisch (DE); Mackiewicz, Monika, 23568 Lübeck (DE)

(56) Entgegenhaltungen:
- WO-A1-2006/034716
- WO-A1-2010/146184
- DE-C1- 4 004 537
- US-A- 4 613 568

## Beschreibung

Die vorliegende Erfindung richtet sich auf ein Verfahren zur Detektion von *Trichinella* in einer Gewebeextraktprobe *Trichinella spiralis (T. spiralis)* ist ein Fadenwurm der Gattung *Trichinella* und kann schwere Erkrankungen beim Menschen auslösen. Die so genannte Trichinellose entsteht durch den Verzehr von rohem oder ungenügend erhitztem Fleisch, das mit Trichinellen infiziert ist. Symptome dieser Erkrankung sind anfänglich Übelkeit, Diarrhö und Muskelschmerz. Mit fortschreitender Erkrankung kommen u.a. Lähmungserscheinungen, Schwellungen im Gesicht - insbesondere periorbital -, Konjunktivitis, Kopfschmerz, Hautauschlag und Myokarditis hinzu [1]. Generell übertragen wird der Parasit durch das Fleisch verschiedener Säugetiere wie Hausschweine, Pferde, Bären, Wildschweine oder Nagetiere. Bekannt sind 11 verschiedene Arten der Gattung *Trichinella,* die sich in zwei Gruppen einteilen lassen: Arten wie *T. spiralis,* die in der Muskelzelle des Wirtsorganismus eine Kollagen-Kapsel bilden von der sie dauerhaft eingeschlossen sind und Arten, die keine Kapsel ausbilden wie *T. pseudospiralis [2].*

*T. spiralis* kommt klimaunabhängig mit wenigen Ausnahmen weltweit vor. In der Europäischen Union (EU) gibt es selten Fälle von Trichinellose bei Hausschweinen, die aus kommerziellen Zucht- und Mastbetrieben stammen, doch bei Wildtieren wie Wildschweinen, Füchsen und Marderhunden oder privat gehaltenen Schweinen liegt die Prävalenz deutlich höher. In China liegt die Prävalenz bei Schweinen generell bei bis zu 4%. Es kam dort zwischen 1964 und 2002 zu über 500 Ausbrüchen mit insgesamt über 25.000 erkrankten Personen [3].

Der Lebenszyklus von *T. spiralis* ist weitgehend bekannt. Nach der oralen Aufnahme der mit *T. spiralis* infizierten Nahrungsmittel werden die Larven nach ca. 24 h im Dünndarm freigesetzt, indem die Kollagen-Kapsel durch die Verdauungsflüssigkeit zersetzt wird. Über vier Häutungen entwickelt sich die Larve zu einer adulten Form und es kommt zur Begattung der Weibchen. Nach 5-10 Tagen werden neue Larven geboren (NBL - *New Born Larvae),* die sich über das Blut und Lymphgefäßsystem verbreiten. 6-12 Tage später dringen die Larven in die quergestreifte Muskulatur ein (ML - Muskellarven) und nach 4-6 Wochen beginnt die Kapselbildung, wobei die Kapsel mit der Zeit zunehmend verkalkt. Über Jahre bis Jahrzehnte findet ein Stoffwechselaustausch mit dem Gewebe statt [4].

Um jahrelang in der Muskulatur des Wirts zu überleben, manipuliert *T. spiralis* das Wirts-Immunsystem mit Hilfe zahlreicher Proteine, die in das umliegenden Gewebe sezerniert werden. Die so genannten exkretorischen und sekretorischen Proteine (E/S-Proteine) werden überwiegend vom Stichosom abgesondert, das aus ca. 50 großen Drüsenzellen, den Stichozyten, besteht und sich in der Ösophaguswand befindet.

Die Trichinenuntersuchung, früher als Trichinenschau bezeichnet, ist eine Untersuchung von Fleisch lebensmittelliefernder Tiere auf *T. spiralis* nach der Schlachtung. Sie ist Teil der amtlichen Schlachttier- und Fleischuntersuchung bei untersuchungspflichtigen Schlachttieren und wurde eingeführt, weil es Mitte des 19. Jahrhunderts zu mehreren Epidemien kam. So wurde bereits 1866 im Königreich Preußen die obligatorische Trichinenschau eingeführt. Wenn das Fleisch zum Verzehr für den Menschen freigegeben werden soll, müssen alle Tiere innerhalb der EU untersucht werden, die Träger von *T. spiralis* sein können wie z.B. Hausschweine, Pferde, Bären oder Wildschweine (EG Nr. 2015/1375).

Die Kosten der Trichinenuntersuchung belaufen sich pro Schwein auf 0,12 € bis 3,70 €, abhängig von der Größe des Schlachthofes und der damit verbundenen Menge an Schlachttieren [5].

Die Serologie ist bei der Trichinenuntersuchung nicht sinnvoll, da eine Serokonversion bei einer Infektionsdosis von 20.000 Larven nach 3-4 Wochen und von 100 Larven nach 5-7 Wochen stattfindet [6].

Es gibt verschiedene Nachweissysteme zur Detektion von *T. spiralis* in Gewebe, die laut EU Verordnung (EG Nr. 2015/1375) zulässig sind und auf Schlachthöfen oder im Labor Anwendung finden. Neben der mechanisch unterstützten Methode der künstlichen Verdauung mit dem Stomacher Lab-blender 3500, dem automatischen Verdauungsverfahren für Sammelproben bis zu 35 g mit dem Trichomatic-35^{®}-Mixer und der Prüfung durch künstliche Digestion mittels des PrioCHECK^{®} Trichinella AAD Kits, gibt es noch zwei weitere Verfahren, nämlich das Magnetrührverfahren für die künstliche Verdauung von Sammelproben und die "On-Filter-Isolation"-Technik mit Larvennachweis mittels eines Latexagglutinationstests (Trichin-L-Antigen-Test-Kit von Bio-Rad). In diesem Zusammenhang soll angemerkt werden, dass das am häufigsten durchgeführte und gleichzeitig als Referenz geltende Verfahren das Magnetrührverfahren für die künstliche Verdauung ist. Die neuste Methode ist das Magnetrührverfahren für die künstliche Verdauung von Sammelproben mit anschließender "On-Filter-Isolation"-Technik und Larvennachweis mittels eines Latexagglutinationstests.

Das Magnetrührverfahren für die künstliche Verdauung von Sammelproben ist dem Fachmann hinreichend bekannt [7]. Dieses Verfahren hat zahlreiche Nachteile, wie eine variierende Qualität des verwendeten Pepsins, Temperatur- und Zeitsensitivität (die Verdauung sollte für 30 Minuten bei 46 °C bis 48 °C erfolgen), hohe Zeitintensität (durch Verdauungsprozess, Sedimentationsschritte, Reinigung der Geräte und Mikroskopie), manuelle Auswertung durch speziell geschultes Personal, schwierige und z.T. gefährliche Handhabung einzelner Schritte durch z.B. den Umgang mit Salzsäure, schwierige Auswertung (z.B. kann die Verdauungsflüssigkeit ungenügend gewaschen worden sein und die Larven können somit durch die zu starke Trübung übersehen werden) und Kontaminationsgefahr durch schlecht gereinigte Gerätschaften.

Auch die "On-Filter-Isolation"-Technik und der anschließende Larvennachweis mittels des Latexagglutinationstests Trichin-L besitzt zahlreiche Nachteile. Beispielsweise weist das hier genannte Verfahren ebenfalls die oben erwähnten Nachteile in Bezug auf die Verdauung auf, da der Verdauungsschritt identisch ist. Ferner kann die Sensitivität des Tests durch chemische Produkte wie Detergenzien in Reinigungslösungen stark beeinträchtigt sein. Zusätzlich gibt es viele Geräte, die gründlich gereinigt werden müssen, wodurch das Risiko einer Kontamination recht hoch ist.

DE4004537 C1 offenbart eine Vorrichtung zur künstlichen Verdauung von Fleisch, wobei das Fleisch chemisch und mechanisch bearbeitet wird.

WO2006/034716 A1 offenbart ein System und Verfahren zum Nachweis von *Trichinella* Larven, wobei Fleisch enzymatisch verdaut, die entstehende Lösung gefiltert und anschließend analysiert wird.

US4613568 A offenbart ein Verfahren zum Herausarbeiten von Parasiten und deren Eier aus Fleisch, wobei Pepsin eingesetzt wird und verschiedene Anpassungen des pH-Werts vorgenommen werden.

WO2010/146184 A1 offenbart Verfahren zur Verdauung von Fleisch mit im basischen Milieu aktiven Verdauungsenzymen.

Daher besteht ein Bedarf an einem Verfahren, das durch einfache Handhabung einen schnellen, preisgünstigen und v.a. zuverlässigen *Trichinella* Nachweis in tierischem Gewebe, insbesondere Muskelgewebe, erlaubt.

Verfahren, Verwendungen und Kits, die einen schnellen, preisgünstigen und zuverlässigen *Trichinella* Nachweis in Gewebe erlauben, werden nachfolgend beschrieben (nur das Verfahren ist Teil der Erfindung)

Die Erfinder der vorliegenden Erfindung haben überraschenderweise gefunden, dass durch eine (mechanische) Zerkleinerung von mit *Trichinella* infiziertem Gewebe ein *Trichinella* Nachweis über einen Immunassay erfolgen kann. Ein derartiger Assay erlaubt die Detektion von ≤ 7 ng *Trichinella* Antigen pro ml Gewebeextrakt oder weniger. Insbesondere konnte gezeigt werden, dass sobald in der Gewebeextraktprobe 90% der darin enthaltenen Partikel einen Durchmesser von 3200 µm oder weniger (D90 ≤ 200 µm) haben, ein effizienter Nachweis von *Trichinella* erfolgen kann.

In einem ersten Aspekt richtet sich die vorliegende Erfindung daher auf ein Verfahren zur Detektion von *Trichinella* in einer Gewebeextraktprobe, wobei in der Gewebeextraktprobe 90% der Partikel einen Durchmesser von 200 µm oder weniger (D90 ≤ 200 µm) haben und wobei bei der Herstellung der Gewebeextraktprobe keine enzymatische und/oder chemische Spaltung des Gewebes erfolgt. Bevorzugt wird *Trichinella* dabei durch Nachweis eines Antigens von Trichinella, bevorzugt eines für *Trichinella* spezifischen Antigens in der Gewebeextraktprobe mittels Immunoassay nachgewiesen.

In bevorzugten Ausführungsformen der Erfindung ist die Gewebeextraktprobe eine Säugetierprobe, bevorzugt eine Probe von einem Schwein.

In weiteren bevorzugten Ausführungsformen stammt die Gewebeextraktprobe aus Muskulatur.

In bevorzugten Ausführungsformen des erfindungsgemäßen Verfahrens wird bei der Herstellung der Gewebeextraktprobe (a) eine Temperatur von 45 °C, bevorzugt 40 °C, nicht überschritten Ferner umfasst das Verfahren in bevorzugten Ausführungsformen (a) keinen Mikroskopierschritt; (b) die Verwendung im Rahmen der Fleischbeschau und/oder (c) besitzt eine Nachweisegrenze von ≤ 7 ng Antigen pro ml Gewebeextrakt.

In weiteren bevorzugten Ausführungsformen wird das erfindungsgemäße Verfahren durch einen Immunassay, weiter bevorzugt durch einen ELISA, Lineblot-Assay, Western Blot-Assay, bead-basierten Assay, Lateral-Flow Assay, vertikalen Filtrationsassay oder 3D-Immunofiltrationsassay durchgeführt.

In bevorzugten Ausführungsformen ist *Trichinella Trichinella spiralis.*

Ferner hierin beschrieben und nicht Teil der Erfindung ist eine Verwendung eines Nachweissystems zur Detektion von *Trichinella,* bevorzugt im Rahmen der Fleischbeschau, wobei das Nachweissystem (a) einen Nachweisträger, der einen ersten Antikörper gegen ein oder mehrere Antigene von *Trichinella* umfasst, und (b)(i) einen zweiten Antikörper gegen ein oder mehrere Antigene von *Trichinella* umfasst, wobei der zweite Antikörper an ein Signalmolekül gebunden ist, oder (b)(ii) ein oder mehrere Antigene von *Trichinella* umfasst, die an ein Signalmolekül gebunden sind, wobei die Antigene aus (b)(ii) so ausgebildet sind, dass sie eine Bindung der Antigene aus (a) an den ersten Antikörper durch kompetitive Verdrängung lösen.

Ferner hierin beschrieben und nicht Teil der Erfindung ist ein Kit umfassend (a) einen Nachweisträger, der einen ersten Antikörper gegen ein oder mehrere Antigene von *Trichinella* umfasst, und (b)(i) einen zweiten Antikörper gegen ein oder mehrere Antigene von *Trichinella* umfasst, wobei der zweite Antikörper an ein Signalmolekül gebunden ist, oder (b)(ii) ein oder mehrere Antigene von *Trichinella* umfasst, die an ein Signalmolekül gebunden sind, wobei die Antigene aus (b)(ii) so ausgebildet sind, dass sie eine Bindung der Antigene aus (a) an den ersten Antikörper durch kompetitive Verdrängung lösen.

Das Kit kann ferner eine Beschreibung für ein erfindungsgemäßes Verfahren zur Detektion von *Trichinella* umfassen.

Wie bereits beschrieben richtet sich der erste Aspekt der vorliegenden Erfindung auf ein Verfahren zur Detektion von *Trichinella* in einer Gewebeextraktprobe, wobei in der Gewebeextraktprobe 90% der Partikel einen Durchmesser von 200 µm oder weniger (D90 ≤ 3200 µm) haben.

Der Ausdruck "Detektion" oder "Detektieren", wie hierin gleichwertig verwendet, beschreibt die qualitative oder quantitative Bestimmung von *Trichinella.* Die qualitative Bestimmung bedeutet, dass lediglich die Anwesenheit oder Abwesenheit von *Trichinella* bestimmt wird. Die quantitative Bestimmung bezieht sich auf die Bestimmung der relativen oder absoluten Menge von *Trichinella* in einer Probe.

Der Ausdruck *"Trichinella"* oder "Trichinen", wie hierin gleichwertig verwendet, bezieht sich auf eine Gattung von Fadenwürmern (Stamm *Nematoda*) mit parasitischer Lebensweise. Säugetiere, und damit auch Menschen, und Vögel dienen als Zwischen- und Endwirt. Hauptüberträger auf den Menschen sind infizierte Schweine bzw. deren rohes, z. B. als Mett verzehrtes, oder ungenügend gegartes Fleisch. Taxonomisch sind Trichinen wie folgt einzuordnen: Stamm: Fadenwürmer (*Nematoda*); Klasse: Adenophorea (*Adenophorea*); Unterklasse: Enoplea (*Enoplea*); Ordnung: *Trichocephalida*; Familie: *Trichinellidae*; Gattung: *Trichinella.* In bevorzugten Ausführungsformen der Erfindung ist *Trichinella* ausgewählt aus der bestehend aus *Trichinella spiralis, Trichinella nativa, Trichinella britovi, Trichinella murrelli, Trichinella T6, Trichinella T7, Trichinella nelsoni, Trichinella T8, Trichinella T9, Trichinella pseudospiralis, Trichinella papuae* und *Trichinella zimbabwensis.* In weiter bevorzugten Ausführungsformen ist die *Trichinella* Art ein Organismus, der in einer Muskelzelle des Wirtsorganismus eine Kollagen-Kapsel bildet und von dieser dauerhaft eingeschlossen ist. In einer noch weiter bevorzugten Ausführungsform ist *Trichinella Trichinella spiralis. Trichinella spiralis* ist ein Fadenwurm und in Mitteleuropa der bedeutendste Vertreter der Trichinen. Er kommt weltweit vor, in tropischen Gebieten hat er aber keine große Bedeutung. *T*. *spiralis* verursacht das klinische Bild der Trichinellose.

Der Ausdruck "Gewebeextraktprobe", wie hierin verwendet, bezeichnet hier eine Mischung aus verschiedenen Substanzen biologischer Herkunft. Bei dem Material biologischer Herkunft kann es sich um Epithelgewebe (Zellschichten, die alle inneren und äußeren Oberflächen bedecken), Binde- und Stützgewebe (Gewebe, das für strukturellen Zusammenhalt sorgt und Zwischenräume füllt), spezialisiertes Gewebe (wie beispielsweise Blut, freie Zellen, etc.), Muskelgewebe (Zellen, die durch kontraktile Filamente für aktive Bewegung spezialisiert sind), Nervengewebe (Zellen, aus denen Gehirn, Rückenmark und periphere Nerven aufgebaut sind) und Gewebsflüssigkeit, wie beispielsweise die Lymphe, handeln. Weiter bevorzugt ist das Gewebe Muskelgewebe. Das Muskelgewebe kann glatte Muskulatur, Herzmuskulatur und/oder Skelettmuskulatur sein. In weiter bevorzugten Ausführungsformen wird die Probe dem Zwerchfell, der Zunge oder der Zwischenrippenmuskulatur des zu untersuchenden Probanden entnommen. Bevorzugt handelt es sich bei dem Gewebe um ein festes Gewebe.

Eine Gewebeextraktprobe kann sowohl heterogen oder auch homogen sein. Eine heterogene Gewebeextraktprobe beinhaltet Gewebe verschiedener Gewebetypen. Eine homogene Gewebeextraktprobe umfasst ausschließlich ein gegebenes Gewebe. Eine homogene Gewebeextraktprobe wird bevorzugt. In alternativen Ausführungsformen ist die Probe eine gepoolte Probe, d.h. dass das Probenmaterial aus verschiedenen Individuen stammt. Oder die Probe stammt ausschließlich von einem einzigen Individuum.

Der Extrakt kann aus Gewebestücken oder lebensfähigen Zellen gewonnen werden. Diese werden zerkleinert und mit einerwässrigen Lösung, wie beispielsweise Pufferlösungen, HzO, Zellmedien und Mischungen davon, versetzt. Bevorzugt umfasst der Herstellungsprozess keine Zellkultivierung.

In bevorzugten Ausführungsformen der Erfindung ist die Gewebeextraktprobe eine Säugetierprobe. In weiter bevorzugten Ausführungsformen stammt die Probe von Schweinen, Pferden, Bären, Katzen, Hunden, Nagetieren oder Menschen. Noch weiter bevorzugt stammt die Probe von einem Hausschwein (*Sus scrofa domesticus*), einem Wildschwein (*Sus scrofa*), Zwergwildschwein (*Sus salvanius*), Bartschwein (*Sus barbatus*), Palawan-Bartschwein (*Sus ahoenobarbus*), Annamitisches Pustelschwein (*Sus bucculentus*), Visayas-Pustelschwein (*Sus cebifrons*), Sulawesi-Pustelschwein (*Sus celebensis*), Mindoro-Pustelschwein (*Sus oliven*), Philippinisches Pustelschwein (*Sus philippensis*), Javanisches Pustelschwein (*Sus verrucosus*) oder Bawean-Pustelschwein (*Sus blouchi*).

Der Ausdruck "Partikeldurchmesser", wie hierin verwendet, bezieht sich auf eine volumetrische oder Längen-Messung der untersuchten Partikel im Gewebeextrakt. Die untersuchten Partikel können eine ungefähr rundliche Form oder eine längliche faserförmige Struktur besitzen. In bevorzugten Ausführungsformen haben in der Gewebeextraktprobe 90% der Partikel einen Durchmesser von 200 µm oder weniger (D90 ≤ 200 µm), 190 µm oder weniger (D90 ≤ 190 µm), 180 µm oder weniger (D90 ≤ 180 µm), 170 µm oder weniger (D90 ≤ 170 µm), 160 µm oder weniger (D90 ≤ 160 µm), 150 µm oder weniger (D90 ≤ 150 µm), 140 µm oder weniger (D90 ≤ 140 µm), 130 µm oder weniger (D90 ≤ 130 µm), 120 µm oder weniger (D90 ≤ 120 µm), 110 µm oder weniger (D90 ≤ 110 µm), 100 µm oder weniger (D90 ≤ 100 µm), 95 µm oder weniger (D90 ≤ 95 µm), 90 µm oder weniger (D90 ≤ 90 µm), 85 µm oder weniger (D90 ≤ 85 µm), 80 µm oder weniger (D90 ≤ 80 µm), 75 µm oder weniger (D90 ≤ 75 µm), 70 µm oder weniger (D90 ≤ 70 µm), 65 µm oder weniger (D90 ≤ 65 µm), 60 µm oder weniger (D90 ≤ 60 µm), 55 µm oder weniger (D90 ≤ 55 µm), 50 µm oder weniger (D90 ≤ 50 µm), 45 µm oder weniger (D90 ≤ 45 µm), 40 µm oder weniger (D90 ≤ 40 µm), 35 µm oder weniger (D90 ≤ 35 µm), 30 µm oder weniger (D90 ≤ 30 µm), 25 µm oder weniger (D90 ≤ 25 µm), 20 µm oder weniger (D90 ≤ 20 µm), 17 µm oder weniger (D90 ≤ 17 µm), 15 µm oder weniger (D90 ≤ 15 µm), 13 µm oder weniger (D90 ≤ 13 µm), 10 µm oder weniger (D90 ≤ 10 µm), 8 µm oder weniger (D90 ≤ 8 µm) oder 6 µm oder weniger (D90 ≤ 6 µm).

Die Messung des Partikeldurchmessers kann durch Geräte, die eine dynamische Bildanalyse verwenden (z.B. CamsizerOXT von der Firma Retsch), oder Geräte, die auf dem Funktionsprinzip der statischen Laserstreuung basieren (z.B. LA-960 von der Firma HORIBA), erfolgen. Die Partikelgröße kann in x_{c min} gemessen werden und ist nach DIN 66141 folgendermaßen definiert: Kürzester Partikeldurchmesser der Messungen der maximalen Durchmesser innerhalb einer Partikelprojektion (englisch: *particle diameter which is the shortest chord of the measured set of maximum chords of a particle projection*) [10]. Alternativ kann die Partikelgröße anhand des Feret-Durchmessers (x_{Fe}) gemessen werden. Der Feret-Durchmesser ist ein Maß für die Objektgröße entlang einer bestimmten Richtung. Im Allgemeinen kann er als der Abstand zwischen den zwei parallelen Ebenen definiert werden, die das Objekt senkrecht zu dieser Richtung einschränken. Man nennt ihn deshalb auch den Kaliberdurchmesser, bezogen auf die Messung der Objektgröße mit einer Schieblehre. Bei der Analyse von Partikelgrößen, beispielsweise in der Mikroskopie, wo der Feret-Durchmesser auf Projektionen eines dreidimensionalen (3D) Objekts auf einer 2D-Ebene angewendet wird, ist dieser definiert als der Abstand zwischen zwei parallelen tangentialen Linien anstelle von zwei Ebenen. Für einen konvexen Partikel ist der mittlere Feret-Durchmesser (Mittelwert aller Richtungen) gleich dem Durchmesser eines Kreises mit gleichem Umfang. Der maximale Feret-Durchmesser ist der längste Feret-Durchmesser innerhalb des gemessenen Satzes von Feret-Durchmessern. Der minimale Feret-Durchmesser ist der kürzeste Feret-Durchmesser innerhalb des gemessenen Satzes von Feret-Durchmessern.

Alternativ bezieht sich der Durchmesser auf einen mittleren Durchmesser, wobei die Summe der Durchmessermessungen aller gemessenen messbaren Partikel, durch die Gesamtzahl der gemessenen Partikel dividiert wird. In einer weiteren alternativen Ausführungsform kann sich der Durchmesser, wenn er in Bezug auf die Größe der Partikel verwendet wird, auf "D50" beziehen, so dass etwa 50% aller gemessenen Partikel einen Partikeldurchmesser aufweisen, der kleiner als der definierte mittlere Partikeldurchmesserwert ist, und dass etwa 50% aller gemessenen messbaren Partikel einen Partikeldurchmesser haben, der größer als der definierte mittlere Partikeldurchmesserwert ist.

In bevorzugten Ausführungsformen des erfindungsgemäßen Verfahrens wird bei der Herstellung der Gewebeextraktprobe (a) eine Temperatur von 100 °C, 90 °C, 80 °C, 70 °C 60 °C, 55 °C, 50 °C, 45 °C, 44 °C, 43 °C, 42 °C 41 °C, 40 °C, 39 °C oder 38 °C nicht überschritten, Der Ausdruck "Herstellung der Gewebeextraktprobe", wie hierin verwendet, bezieht sich auf einen mehrschrittigen Vorgang, wobei eine Gewebeprobe aus einem zu untersuchenden Organismus entnommen, diese Gewebeprobe (mechanisch) zerkleinert und die zerkleinerte Gewebeprobe durch Filtration und/oder Zentrifugation behandelt wird. Anschließend kann der Antigen-Nachweis im erfindungsgemäßen Verfahren erfolgen.

Die Zerkleinerung der Gewebeprobe erfolgt rein mechanisch, d.h. beispielsweise keine enzymatische und/oder chemische Spaltung des Gewebes. Die mechanische Zerkleinerung kann über Schneiden, Zerreißen oder Zerquetschen erfolgen, wird bevorzugt aber durch Schneiden (z.B. über eine Messermühle) erreicht. Durch die Zerkleinerung der Gewebeprobe werden ebenfalls die in der Gewebeprobe befindlichen *Trichinella* Larven zerkleinert. Die zerkleinerten *Trichinella* Larven haben nachdem zerkleinern eine Größe, die dem zerkleinerten Gewebe entspricht.

Der Ausdruck "keine enzymatische Spaltung", wie hierin verwendet, bezieht sich darauf, dass zur Zerkleinerung der Gewebeprobe keine Enzyme eingesetzt werden. Insbesondere werden zur Zerkleinerung der Gewebeprobe keine Proteasen (z.B. Pepsin), Lipasen, Amylasen, Cutinasen, Cellulasen oder Hemicellulasen verwendet.

Der Ausdruck "keine chemische Spaltung", wie hierin verwendet, bezieht sich darauf, dass zur Zerkleinerung der Gewebeprobe keine chemischen Substanzen, wie Säuren, Basen, Oxidationsmittel usw., eingesetzt werden.

Ferner umfasst das Verfahren in bevorzugten Ausführungsformen (a) keinen Mikroskopierschritt; (b) die Verwendung im Rahmen der Fleischbeschau und/oder (c) besitzt eine Nachweisegrenze von < 20 ng, < 15 ng, < 10 ng, < 9 ng, < 8 ng, < 7 ng, < 6 ng, < 5 ng, < 4 ng, < 3 ng, < 2 ng, < 1 ng, < 0,5 ng, < 0,25 ng, < 0,1 ng, < 0,05 ng, < 0,01 ng, < 0,005 ng oder < 0,001 ng Antigen pro ml Gewebeextrakt.

Der Ausdruck "kein Mikroskopierschritt", wie hierin verwendet, bezieht sich auf die Auswertung eines Verfahrens zum *Trichinella* Nachweis, wobei zur Auswertung des erfindungsgemäßen Verfahrens kein Mikroskop oder eine mikroskopische Bewertung, insbesondere eine manuelle mikroskopische Bewertung, notwendig ist.

Der Ausdruck "Fleischbeschau" oder auch "Schlachttier- und Fleischuntersuchung", wie hierin verwendet, bezieht sich auf ein Verfahren, durch das sichergestellt werden soll, dass das Fleisch bestimmter Tierarten als Lebensmittel nur in den Verkehr gelangt, wenn es als tauglich zum Genuss für Menschen beurteilt worden ist. Diese Untersuchung ist ein wesentlicher Bestandteil der Maßnahmen zur Gewährleistung der Fleischhygiene. Die Untersuchung erfolgt in der Regel durch amtliche Tierärzte oder Fleischkontrolleure in zwei Schritten, nämlich der Untersuchung des Tieres, und der Untersuchung des Fleisches.

In einer bevorzugten Ausführungsform gibt der Ausdruck "Nachweisgrenze", wie hierin verwendet, die geringste Menge einer Substanz (Antigen) an, die von der Abwesenheit dieser Substanz mit einer spezifischen Wahrscheinlichkeit unterschieden werden kann. Alternativ kann sich der Begriff "Nachweisgrenze" auf die Konzentration eines Antigens in einer Lösung beziehen, bei der der gemessene Wert größer ist als die damit verbundene Unsicherheit. Die Nachweisgrenze kann willkürlich als 3 Standardabweichungen (SD) entfernt von der Nullkonzentration definiert werden.

In weiteren bevorzugten Ausführungsformen wird das erfindungsgemäße Verfahren durch einen Immunassay, weiter bevorzugt durch einen ELISA, Lineblot-Assay, Western Blot-Assay, bead-basierten Assay, Lateral-Flow Assay, vertikalen Filtrationsassay oder 3D-Immunofiltrationsassay, durchgeführt.

In einer bevorzugten Ausführungsform bezieht sich der Ausdruck "Immunassay", wie hierin verwendet, auf die Detektion oder Quantifizierung eines Analyten - wie eines gegebenen Antigens von *Trichinella* -, umfassend eine Immunreaktion zwischen einem Antikörper und dem Antigen. Im Rahmen der Erfindung kann der nachzuweisende oder zu quantifizierende Analyt ein Peptid, ein posttranslational modifiziertes Peptid, bevorzugt ein Glykoprotein, ein Zucker, ein Lipid, eine Nukleinsäure und/oder ein anderes Molekül von *Trichinella* umfassen.

In einer bevorzugten Ausführungsform steht der Ausdruck "ELISA", wie hierin verwendet, für *Enzyme-linked Immunosorbent Assay* und bezieht sich auf ein antikörperbasiertes Nachweisverfahren (Assay). Der ELISA gehört zur Gruppe der Immunassay-Verfahren, basiert auf einer enzymatischen Farbreaktion und zählt somit zu den enzymatischen Immunadsorptionsverfahren (EIA). Bevorzugte Ausführungsformen umfassen direkten ELISA, indirekten ELISA, direkten Sandwich-ELISA, bridging ELISA, indirekten Sandwich-ELISA und kompetitiven ELISA. Dem Fachmann sind die genannten und weitere Formen und Ableitungen von ELISA bekannt.

In einer bevorzugten Ausführungsform ist der Ausdruck "Lateral-Flow-Assay", wie hierin verwendet, (englisch für "seitlicher Flusstest") eine biochemische Methode zum qualitativen Nachweis von Stoffen/Substanzen/Antigenen mit Antikörpern. Der Lateral-Flow-Assay ist eine Kombination aus einer Dünnschichtchromatographie und einer Immunfärbung. Der Lateral-Flow-Assay kann in Form eines Teststreifens verwendet werden.

In einer bevorzugten Ausführungsform beruht der Ausdruck "vertikaler Filtrationsassay", wie hierin verwendet, darauf, dass zu untersuchende Liganden/Antigene mit einer Membran kontaktiert werden, an der Fängerantikörper immobilisiert sind. Es schließt sich ein Waschvorgang zur Entfernung schwach gebundener Moleküle und ein Nachweis der gebundenen Liganden an. Der Unterschied zwischen Lateral-Flow-Assay und vertikalem Filtrationsassay ist der der laterale und der vertikale Fluss der Test-Flüssigkeit. Die vertikale Strömungstechnologie hat einige Vorteile gegenüber dem Lateral-Flow-Assay, wobei z.B. kürzere Assayzeiten vorkommen können.

In einer bevorzugten Ausführungsform bezieht sich der Ausdruck "3D-Immunofiltrationsassay", wie hierin verwendet, auf einen immunologischen Schnelltest im Durchfluss-Assayformat, der auf dem gleichen biochemischen Funktionsprinzip der Analyterkennung durch Rezeptorstrukturen basiert wie der Lateral-Flow-Assay. Der Unterschied besteht darin, dass die Zugabe der Probe/Antigen-, Konjugat- und zusätzlichen Waschlösungen sequentiell auf einen dreidimensionalen porösen Formkörper erfolgt, an den Fängerantikörper immobilisiert sind. Alle Lösungen und deren Bestandteile wie Analyten/Antigene und Detektionsreagenzien fließen dabei im Durchfluss in die Tiefe des Formkörpers. Unter Ausnutzung von Anreicherungseffekten ist es möglich, Nachweisgrenzen zu erhöhen.

In einer bevorzugten Ausführungsform bezieht sich der Ausdruck "Lineblot" auf einen Teststreifen, auf den wenigstens ein gereinigtes Antigen auf einer genau vorbestimmten Position auf dem Streifen durch Aufdrucken aufgebracht ist. Die Herstellung solcher Teststreifen ist im Stand der Technik beschrieben. Ist in der Probe Antikörper vorhanden, so kann dessen Komplex mit dem Antigen kolorimetrisch nachgewiesen werden. Die Auslesung erfolgt visuell oder durch Intensitätsmessung entstehender Banden. Der Teststreifen kann eine Positivkontrolle in Form einer Bande enthalten, die erscheint, wenn der Streifen mit Serum inkubiert wurde, unabhängig davon, ob dieses den nachzuweisenden Analyten enthält.

In einer bevorzugten Ausführungsform bezieht sich der Ausdruck "bead-basierter Assay" auf einen Test, bei dem als Träger ein Bead, bevorzugt ein magnetischer Bead, eingesetzt wird, auf dem ein Reagenz für den Nachweis, bevorzugt ein Antikörper gegen ein Antigen von Trichinella, immobilisiert ist. Der Nachweis des Antikörper-Antigen-Komplexes kann durch Chemilumineszenz erfolgen, bevorzugt mittels eines zweiten Antikörpers, der ein mittels Chemilumineszenz detektierbares Signalmolekül trägt. Das Bead ist bevorzugt chemisch inert und umfasst reaktionsträge Kohlenhydrate.

In bevorzugten Ausführungsformen des erfindungsgemäßen Verfahrens umfasst dieses Verfahren die folgenden Schritte:
(a) Bereitstellen eines Nachweisträgers, der einen ersten Antikörper gegen ein oder mehrere Antigene von *Trichinella* umfasst;
(b) Kontaktieren des Nachweisträgers mit der Probe;
(c)(i) Kontaktieren des Nachweisträgers und gegebenenfalls daran gebundenes Probenmaterial mit einem zweiten Antikörper gegen ein oder mehrere Antigene von *Trichinella,* wobei der zweite Antikörper an ein Signalmolekül gebunden ist und wobei die Anwesenheit eines Signals des Signalmoleküls die Anwesenheit von *Trichinella* in der Probe anzeigt; oder
(c)(ii) Kontaktieren des Nachweisträgers und gegebenenfalls daran gebundenes Probenmaterial mit einem oder mehreren Antigenen von *Trichinella,* wobei die Antigene aus (c)(ii) an ein Signalmolekül gebunden sind und so ausgebildet sind, dass sie eine Bindung der Antigene aus (a) an den ersten Antikörper durch kompetitive Verdrängung lösen, wobei die Anwesenheit eines Signals des Signalmoleküls die Anwesenheit von *Trichinella* in der Probe anzeigt.

In weiter bevorzugten Ausführungsformen erfolgt jeweils ein Waschschritt nach den Kontaktierschritten (b), (c)(i) und (c)(ii).

Ferner ist in bevorzugten Ausführungsformen des Verfahrens, der Verwendung und des Kits das Signalmolekül unter Verwendung analytischer Techniken, wie Fluoreszenzmessung, Chemilumineszenzmessung, Radioaktivitätsmessung, Elektronenspinresonanzmessung, Ultraviolett/sichtbare Absorptionsspektroskopie, Massenspektrometrie, Kernspinresonanz-, Magnetresonanz- und elektrochemische Messmethoden beobachtbar.

Geeignete Antigene von *Trichinella* sind aus dem Stand der Technik bekannt. Bevorzugt handelt es sich bei dem Antigen um Tyvelose.

In bevorzugten Ausführungsformen richten sich der erste und zweite Antikörper gegen verschiedene Epitope desselben Antigens.

In weiteren bevorzugten Ausführungsformen sind der erste und/oder zweite Antikörper aus der Gruppe bestehend aus einem Antikörper, der sich gegen eine (ggf. an ein Substratprotein gebundene) posttranslationale Modifikation, bevorzugt ein Tyvelose umfassendes Molekül, richtet, einem Antikörper, der sich gegen ein exkretorisches-sekretorisches (E/S)-Antigen von *Trichinella* richtet, und einem Antikörper, der durch Verwendung eines Lysats von *Trichinella spiralis* hergestellt wurde, ausgewählt.

Der Ausdruck "Antikörper", wie hierin verwendet, bezieht sich auf Proteine aus der Klasse der Globuline, die in Wirbeltieren als Reaktion auf bestimmte Stoffe, so genannte Antigene, gebildet werden. Antikörper sind Bestandteile des Immunsystems. Antikörper werden von einer Klasse weißer Blutzellen, den B-Lymphozyten, produziert. Sie können anhand verschiedener Klassen unterschieden werden, nämlich Immunglobulin A, Immunglobulin D, Immunglobulin E, Immunglobulin G, Immunglobulin M, Immunglobulin W und Immunglobulin Y. In bevorzugten Ausführungsformen sind der erste und/oder der zweite Antikörper Immunglobulin G.

In bevorzugten Ausführungsformen sind die verwendeten Antikörper ausgewählt aus der Gruppe bestehend aus einem Antikörper umfassend eine VH Sequenz gemäß SEQ ID NO:1 und eine VL Sequenz gemäß SEQ ID NO:2, einem Antikörper umfassend eine VH Sequenz gemäß SEQ ID NO:3 und eine VL Sequenz gemäß SEQ ID NO:4, Antikörper 18H1(IgG2a), der Tyvelose bindet und in [11] beschrieben ist, und Varianten davon.

Der Ausdruck "Variante", wie hierin verwendet, bezieht sich auf Antikörper und VH- und VL Sequenzen, die mindestens 70%, mindestens 75%, mindestens 80%, mindestens 85%, mindestens 90%, mindestens 95%, mindestens 97% oder mindestens 99% Homologie zum Referenz-Antikörper bzw. zur Referenz-Sequenz besitzen. Bevorzugt werden nur solche Varianten eingesetzt, die biologische Aktivität aufweisen. "Biologische Aktivität", wie in diesem Zusammenhang verwendet, bedeutet insbesondere, dass die entsprechenden Peptide mindestens 30 %, mindestens 40 %, mindestens 50 %, mindestens 60 %, mindestens 70 %, mindestens 80 %, mindestens 90 %, mindestens 95 % der spezifischen Bindeaktivität ihres Referenz-Antikörpers bzw. der Referenz-Sequenz aufweisen. Es können funktionelle Fragmente oder Derivate von Antikörpern oder Varianten davon verwendet werden, beispielsweise Fab, F(ab'), Fr, ScTv und dAb oder Aptamere mit entsprechender Bindeaktivität.

Die Zerkleinerung der Gewebeextraktprobe wird ausschließlich mechanisch durchgeführt. D.h. es gibt keinen Zerkleinerungsschritt, der chemisch und/oder enzymatisch durch von extern dem Verfahren zugefügten Molekülen oder Verbindungen ausgeführt wird. In anderen bevorzugten Ausführungsformen werden dem Verfahren keine externen zusätzlichen chemischen und/oder enzymatischen Moleküle in einer Menge zugesetzt, so dass die proteolytische Aktivität mehr als das vierfache, mehr als das dreifache oder mehr als das doppelte der proteolytischen Hintergrundaktivität erreicht.

In einer weiteren bevorzugten Ausführungsform des erfindungsgemäßen Verfahrens sind in der Gewebeextraktprobe mindestens 30%, mindestens 35%, mindestens 40%, mindestens 45%, mindestens 50%, mindestens 55%, mindestens 60%, mindestens 65%, mindestens 70%, mindestens 75%, mindestens 80%, mindestens 85%, mindestens 90%, mindestens 95%, mindestens 97% oder mindestens 99% aller Proteaseschnittstellen nicht gespalten. Diese Proteasen besitzen eine hydrolytische Aktivität gegenüber Peptidbindungen und gehören der EC-Klasse 3.4. an. In anderen bevorzugten Ausführungsformen des erfindungsgemäßen Verfahrens sind in der Gewebeextraktprobe mindestens 30%, mindestens 35%, mindestens 40%, mindestens 45%, mindestens 50%, mindestens 55%, mindestens 60%, mindestens 65%, mindestens 70%, mindestens 75%, mindestens 80%, mindestens 85%, mindestens 90%, mindestens 95%, mindestens 97% oder mindestens 99% Schnittstellen von Pepsin, Chymosin, Cathepsin E, Papain, Cathepsin K, Caspase, Calpain, Scytalidoglutamische Peptidase, Thermolysin, Kollagenasen, Carboxypeptidase A u. B, Chymotrypsin, Plasmin, Thrombin, Trypsin, Granzyme und/oder Kallikrein nicht gespalten.

In einer weiteren bevorzugten Ausführungsform des erfindungsgemäßen Verfahrens sind in der Gewebeextraktprobe mindestens 30%, mindestens 35%, mindestens 40%, mindestens 45%, mindestens 50%, mindestens 55%, mindestens 60%, mindestens 65%, mindestens 70%, mindestens 75%, mindestens 80%, mindestens 85%, mindestens 90%, mindestens 95%, mindestens 97% oder mindestens 99% Schnittstellen von Pepsin, Chymosin, Chymotrypsin und/oder Trypsin nicht gespalten. Insbesondere sind in der Gewebeextraktprobe mindestens 30%, mindestens 35%, mindestens 40%, mindestens 45%, mindestens 50%, mindestens 55%, mindestens 60%, mindestens 65%, mindestens 70%, mindestens 75%, mindestens 80%, mindestens 85%, mindestens 90%, mindestens 95%, mindestens 97% oder mindestens 99% Schnittstellen von Pepsin nicht gespalten.

Die Schnittstellen der oben genannten Peptidasen sind aus dem Stand der Technik, wie beispielsweise https://web.expasy.org/peptide cutter/peptidecutter enzymes.html#Peps, hinreichend bekannt und sind hiermit durch Verweis in die Beschreibung aufgenommen.

Die Bestimmung der Identität/Homologie von Nukleinsäure- oder Aminosäuresequenzen erfolgt durch einen Sequenzvergleich. Dieser Sequenzvergleich basiert auf dem im Stand der Technik etablierten und üblicherweise genutzten BLAST-Algorithmus (vgl. [12] und [13]) und geschieht prinzipiell dadurch, dass ähnliche Abfolgen von Nukleotiden oder Aminosäuren in den Nukleinsäure- oder Aminosäuresequenzen einander zugeordnet werden. Eine tabellarische Zuordnung der betreffenden Positionen wird als Alignment bezeichnet. Ein weiterer im Stand der Technik verfügbarer Algorithmus istderFASTA-Algorithmus. Sequenzvergleiche (Alignments), insbesondere multiple Sequenzvergleiche, werden mit Computerprogrammen erstellt. Häufig genutzt werden beispielsweise die Clustal-Serie (vgl. beispielsweise [14]), T-Coffee (vgl. beispielsweise [15]) oder Programme, die auf diesen Programmen beziehungsweise Algorithmen basieren. Ferner möglich sind Sequenzvergleiche (Alignments) mit dem Computer- Programm Vector NTI^{®} Suite 10.3 (Invitrogen Corporation, 1600 Faraday Avenue, Carlsbad, Kalifornien, USA) mit den vorgegebenen Standardparametern, dessen AlignX-Modul für die Sequenzvergleiche auf ClustalW basiert.

Solch ein Vergleich erlaubt auch eine Aussage über die Ähnlichkeit der verglichenen Sequenzen zueinander. Sie wird üblicherweise in Prozent Identität, das heißt dem Anteil der identischen Nukleotide oder Aminosäurereste an denselben oder in einem Alignment einander entsprechenden Positionen angegeben. Der weiter gefasste Begriff der Homologie bezieht bei Aminosäuresequenzen konservierte Aminosäure-Austausche in die Betrachtung mit ein, also Aminosäuren mit ähnlicher chemischer Aktivität, da diese innerhalb des Proteins meist ähnliche chemische Aktivitäten ausüben. Daher kann die Ähnlichkeit der verglichenen Sequenzen auch Prozent Homologie oder Prozent Ähnlichkeit angegeben sein. Identitäts- und/oder Homologieangaben können über ganze Polypeptide oder Gene oder nur über einzelne Bereiche getroffen werden. Homologe oder identische Bereiche von verschiedenen Nukleinsäure- oder Aminosäuresequenzen sind daher durch Übereinstimmungen in den Sequenzen definiert. Solche Bereiche weisen oftmals identische Funktionen auf. Sie können klein sein und nur wenige Nukleotide oder Aminosäuren umfassen. Oftmals üben solche kleinen Bereiche für die Gesamtaktivität des Proteins essentielle Funktionen aus. Es kann daher sinnvoll sein, Sequenzübereinstimmungen nur auf einzelne, gegebenenfalls kleine Bereiche zu beziehen. Soweit nicht anders angegeben beziehen sich Identitäts- oder Homologieangaben in der vorliegenden Anmeldung aber auf die Gesamtlänge der jeweils angegebenen Nukleinsäure- oder Aminosäuresäuresequenz.

Der Ausdruck "so ausgebildet, dass eine Bindung der Antigene an den Antikörper durch kompetitive Verdrängung gelöst wird", wie hierin verwendet, bezieht sich auf Antigene, die mit bereits gebundenen Antigenen um die Bindung an einen gegebenen Antikörper konkurrieren. Die kompetitiven Antigene haben eine strukturelle Ähnlichkeit zueinander und daher können die Antigene als Strukturanaloga bezeichnet werden. Das verdrängende Antigen kann eine höhere Affinität an den Antikörper besitzen als das verdrängte Antigen und/oder das verdrängende Antigen ist in höherer Konzentration vorhanden als das verdrängte Antigen.

Der Ausdruck "Antigen", wie hierin verwendet, bezieht sich bevorzugt auf Stoffe, an die sich Antikörper und bestimmte Lymphozyten-Rezeptoren spezifisch binden können. Antigene können Proteine, aber auch Glykoproteine, Kohlenhydrate, Lipide oder andere Stoffe sein. Vorliegend sind die Antigene bevorzugt Proteine oder posttranslational modifizierte Proteine.

Ferner hierin beschrieben und nicht Teil der Erfindung ist eine Verwendung eines Nachweissystems zur Detektion von *Trichinella,* bevorzugt zur Fleischbeschau, wobei das Nachweissystem (a) einen Nachweisträger, der einen ersten Antikörper gegen ein oder mehrere Antigene von *Trichinella* umfasst, und (b)(i) einen zweiten Antikörper gegen ein oder mehrere Antigene von *Trichinella* umfasst, wobei der zweite Antikörper an ein Signalmolekül gebunden ist, oder (b)(ii) ein oder mehrere Antigene von *Trichinella* umfasst, die an ein Signalmolekül gebunden sind, wobei die Antigene aus (b)(ii) so ausgebildet sind, dass sie eine Bindung der Antigene aus (a) an den ersten Antikörper durch kompetitive Verdrängung lösen.

Der Ausdruck "Nachweissystem", wie hierin verwendet, umfasst bevorzugt (a) einen Nachweisträger wie hierin definiert und (b) ein/einen an ein Signalmolekül gebundenes(n) Antigen oder Antikörper. Die Komponenten aus (a) und (b) wirken derart synergistisch zusammen, dass durch die Zugabe eines zu untersuchenden Antigens ein Bindungskomplex aus allen beteiligten Molekülen entsteht, der über die Anwesenheit eines Signals den Nachweis des zu untersuchenden Antigens in einer Probe erlaubt.

Der Ausdruck "Nachweisträger" wie hierin verwendet, bezieht sich bevorzugt auf einen Stoff, an den ein Antikörper gegen ein oder mehrere Antigene von *Trichinella* gebunden ist. Der Träger kann ein fester Gegenstand, wie ein Objektträger, eine 6-Well-, 12-Well-, 96-Well- oder 384-Well-Platte, eine Membran, bevorzugt eine Nitrocellulose-Membran, ein Filtermaterial, ein Bead, bevorzugt magnetischer Bead oder ein Dünnschichtchromatographiematerial sein. Alternativ kann der Nachweisträger auch ein Kügelchen/Bead sein, wobei der Durchmesser eines solchen Beads bevorzugt kleiner als 1000 µm, 800 µm, 600 µm, 400 µm, 200 µm, 100 µm, 90 µm, 80 µm, 70 µm, 60 µm, 50 µm, 40 µm, 30 µm, 20 µm, 10 µm oder 5 µm ist. Der Nachweisträger kann bevorzugt Kunststoff, Glas, Metall oder eine Kombination davon umfassen.

Ferner hierin beschrieben und nicht Teil der Erfindung ist ein Kit umfassend (a) einen Nachweisträger, der einen ersten Antikörper gegen ein oder mehrere Antigene von *Trichinella* umfasst, und (b)(i) einen zweiten Antikörper gegen ein oder mehrere Antigene von *Trichinella* umfasst, wobei der zweite Antikörper an ein Signalmolekül gebunden ist, oder (b)(ii) ein oder mehrere Antigene von *Trichinella* umfasst, die an ein Signalmolekül gebunden sind, wobei die Antigene aus (b)(ii) so ausgebildet sind, dass sie eine Bindung der Antigene aus (a) an den ersten Antikörper durch kompetitive Verdrängung lösen.

Der Ausdruck "Kit", wie hierin verwendet, bezieht sich bevorzugt auf eine Verpackung, die mit Behältern (z. B. Flaschen, Platten, Röhrchen, Schalen usw.) versehen ist, die jeweils ein spezifisches Material enthalten, vorliegend insbesondere einen wie hierin definierten Nachweisträger und ein/einen an ein Signalmolekül gebundenes(n) Antigen oder Antikörper. Vorzugsweise wird dem Kit eine Gebrauchsanweisung zur Verwendung des oben genannten Materials beigelegt. Die Bedienungsanleitung kann auf Papier oder einem anderen Medium geschrieben oder gedruckt sein, oder kann in Form von elektronischen Medien, wie Magnetband, computerlesbarer Disk oder Band oder CD-ROM, bereitgestellt werden. Der Kit enthält bevorzugt eine Positivkontrolle, bevorzugt mit einem nachzuweisenden Antigen und/oder Proben zur Kalibration oder Erstellung einer Kalibrationskurve.

Das Kit kann ferner eine Beschreibung für ein erfindungsgemäßes Verfahren zur Detektion von *Trichinella* umfassen.

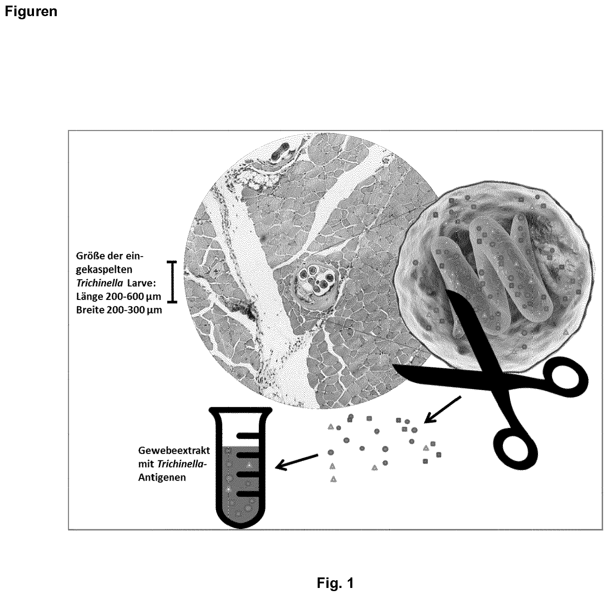

**Fig. 1** zeigt die E/S-Proteine einer eingekapselten *Trichinella spiralis* Larve. Die E/S-Proteine werden von der Larve in die Kapsel und in das umliegende Gewebe abgesondert. Durch die Zerkleinerung des Gewebes werden die Proteine in das Probenmaterial freigesetzt.

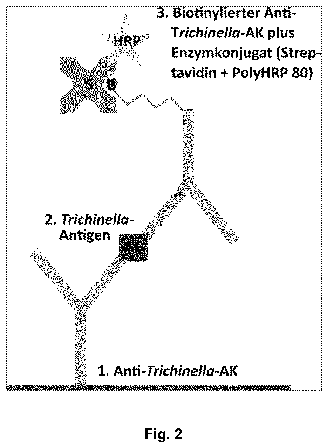

**Fig. 2** zeigt ein Inkubationsschema des *Trichinella Antigen-Capture ELISA.* 1) Auf einer Mikrotiterplatte immobilisierter *Anti-Trichinella-AK* C9, 2) Probe (*Trichinella-Antigen*), 3) Biotinylierter Nachweis-AK B7, gebunden an Streptavidin mit PolyHRP80.

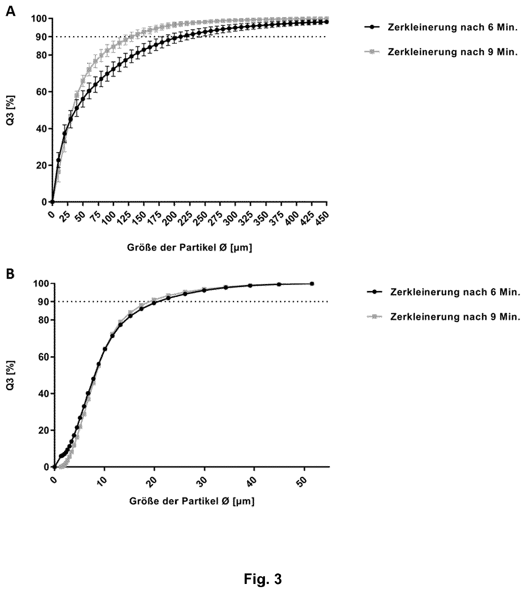

**Fig. 3** zeigt die Partikelgrößenbestimmung des mit der Messermühle GM200 von Retsch zerkleinerten Schweinefleisches. 100 x 1 g Zwerchfellpfeiler-Muskulatur wurden mit 200 ml PBS in der GM200 bei 10.000 rpm für 6 bzw. 9 Min. zerkleinert. Es sind die Partikelgröße gegen Q3[%] (Volumenprozent) aufgetragen. Vermessen wurden jeweils ca. 60. Mio. Partikel. A) Partikelgrößenbestimmung mit dem Camsize^{®}XT. n=3. B) Partikelgrößenbestimmung mit dem HORIBA LA-960. n=1.

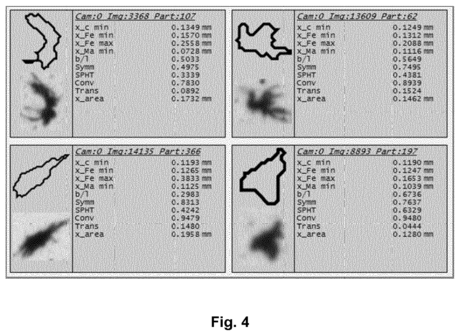

**Fig. 4** zeigt eine Aufnahme einzelner Fleischpartikel während der Messung mit dem Camsizer^{®}XT. Die Form ist meist nicht rund, sondern sehr faserig und langgezogen, sodass das Breite zu Längenverhältnis sinkt.

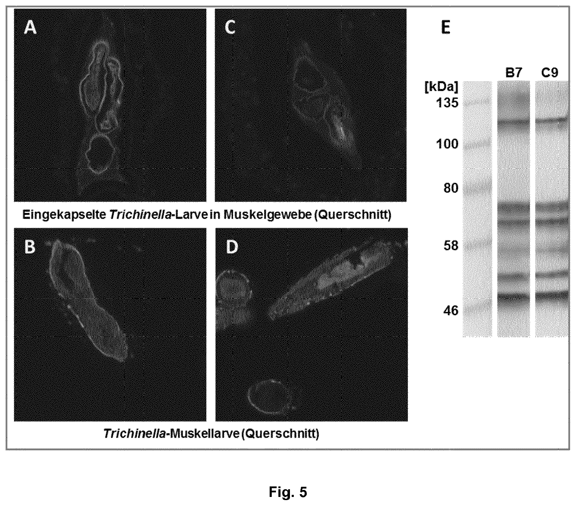

**Fig. 5** zeigt die Prüfung der Antikörper Anti-[TRISP][B7] und Anti-[TRISP][C9] zum Nachweis von *Trichinella.* A)-D) *T. spiralis-*IIFT (Indirekter Immunfluoreszenztest): Die AK B7 und AK C9 wurden mit einer Konzentration von 2 µg/ml inkubiert. A)+B) Inkubation mit AK B7: Der AK zeigt sowohl bei der eingekapselten *Trichinella*-Larve im Querschnitt als auch bei der Muskellarve eine deutliche Reaktion; C)+D) Inkubation mit AK C9: Der AK zeigt wie B7 sowohl bei der eingekapselten *Trichinella*-Larve im Querschnitt als auch bei der Muskellarve eine deutliche Reaktion; E) Westernblot: Aufgetragen sind jeweils 5 µg *T. spiralis* Lysat und die Inkubation erfolgte mit den AK B7 und AK C9 (Konz. 0,4 µg/ml).

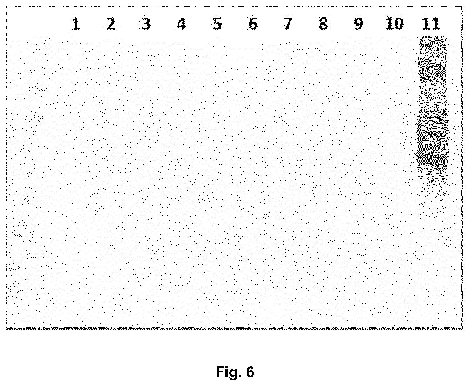

**Fig. 6** zeigt die Prüfung der Spezifität der Antikörper Anti-TRISP[B7] und Anti-TRISP[C9] mittels Westernblot. Spur 1) *Trypanosoma cruzi,* 2) *Ascaris suum,* 3) *Strongyloides ratti,* 4) *Toxocara cati, 5) Toxoplasma gondii,* 6) *Salmonella typhimurium,* 7) *Salmonella cholerasuis* Stamm A, 8) *Salmonella cholerasuis* Stamm B, 9) *Salmonella typhisuis,* 10) *Trichuris suis,* 11) *Trichinella spiralis.* Aufgetragen: Je 5 µg Lysat. Inkubation mit AK B7 (Konz. 0,4 µg/ml, 2 h).

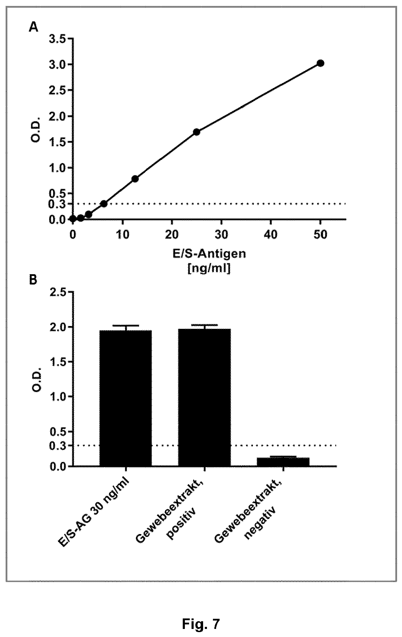

**Fig. 7** zeigt Ergebnisse des *T. spiralis Antigen-Capture ELISA.* A) Detektionsgrenze des *T. spiralis Antigen-Capture ELISA.* Aufgetragen sind verschiedene Konzentrationen an E/S-Antigen gegen die O.D. B) *T. spiralis Antigen-Capture ELISA* mit E/S-Antigen, positivem und negativem Gewebeextrakt als Probenmaterial. In der positiven Gewebeextraktprobe befanden sich ca. 100 eingekapselte *Trichinella-Larven.* Gestrichelte Linie: *Cut-off.*

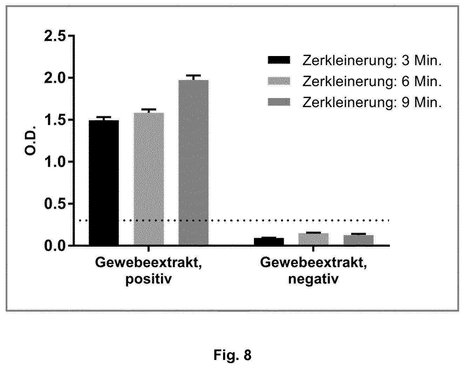

**Fig. 8** zeigt Ergebnisse des *T. spiralis Antigen-Capture ELISA* mit positivem und negativem Gewebeextrakt als Probenmaterial. In dem positiven Gewebeextrakt befanden sich ca. 100 eingekapselte *Trichinella-Larven.* Die Zerkleinerung des Probenmaterials erfolgte für 3, 6 und 9 Min. Gestrichelte Linie: *Cut-off.* n=3.

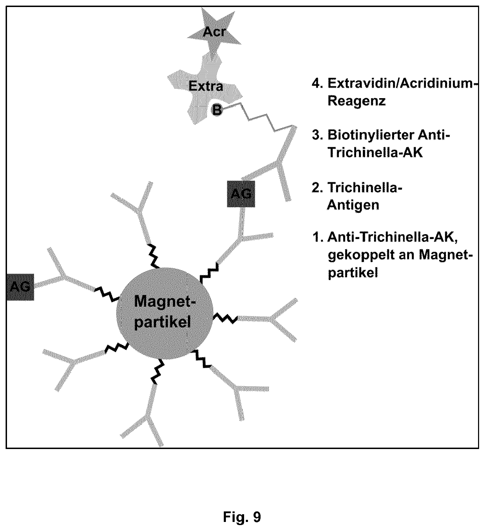

**Fig. 9** zeigt ein Inkubationsschema des manuell inkubierten Trichinella-Chemilumineszenz Immunassay. 1) Auf einem magnetischen Bead immobilisierter Anti-Trichinella-Fänger-AK 18H1, 2) Probe (Trichinella-Antigen), 3) Biotinylierter Anti-Trichinella-Nachweis-AK B7, 4) Extravidin/Acridinium-Reagenz.

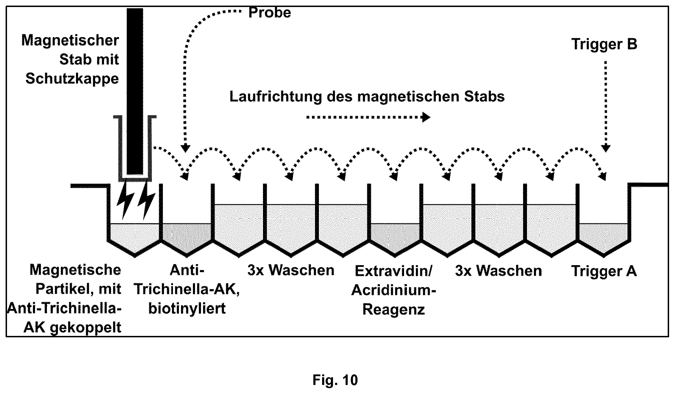

**Fig. 10** zeigt das Abarbeitungsschema des Chemilumineszenz Analysegerätes für den automatisierten Trichinella-Chemilumineszenz Immunassay. Der Magnetstab transportiert die mit Anti-Trichinella-AK 18H1 immobilisierten Beads sukzessive in mit verschiedenen Reagenzien gefüllte Reaktionsgefäße. Die Probe und Trigger B werden automatisiert hinzugegeben.

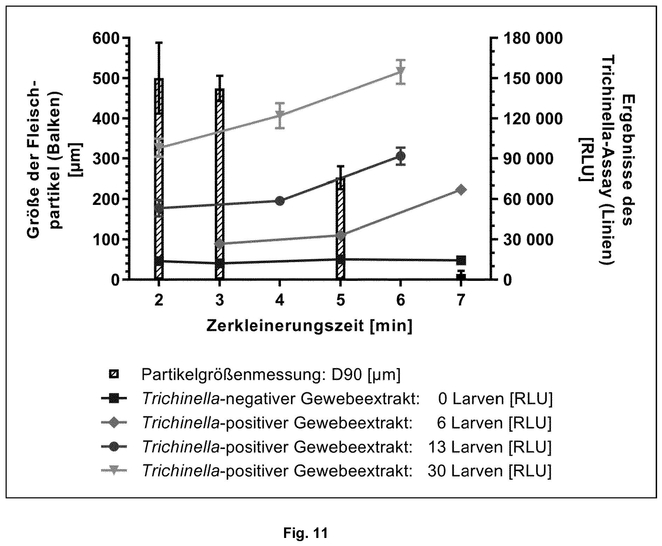

**Fig. 11** zeigt Ergebnisse des automatisierten Trichinella-Chemilumineszenz Immunassays. Die Partikelgrößen des zerkleinerten Schweinefleisches wurden nach 2, 3, 5 und 7 Min. mit dem Partikelgrößenanalysator LA-960 von der Firma HORIBA gemessen. Außerdem wurden Fleischproben - negativ und versetzt mit 6, 13 und 30 Trichinella-Larven - zwischen 2 und 7 Min. zerkleinert. Der entstandene Gewebeextrakt wurde mit dem automatisierten CLlA inkubiert. Die Ergebnisse sind in relativen Lichteinheiten (RLU) angegeben.

### Sequenzen:

In der vorliegenden Erfindung werden verschiedene Peptid-Sequenzen offenbart, insbesondere
**SEQ ID NO:1** (Anti-[TRISP][B7] VH): 
AVTLDESGGGLQTPRGGLSLVCKASGYTFSSHNMAWVRQAPGKGLEFVAGISNTGSFTLYGAAV
KGRATISRDNGQSTVRLQLNNLRAEDTGTYYCAKHAGVGLYSIDAWGHGTEVIVSS
**SEQ ID NO:2** (Anti-[TRISP][B7] VL): 
ALTQPSSVSANLGGTVKITCSGGTSDYGWYQQKAPGSAPVTLIYDNTNRPSDIPSRFSGSLSGST
NTLTITGVQAEDEAVYFCGSADRTYAGVFGAGTTLTVL
**SEQ ID NO:3** (ANTI-[TRISP][C9] VH): 
AVTLDESGGGLQTPGGALSLVCKASGFSISSYSMQWVRQAPGKGLEWVAGIYYDGNTWYAPAVK
GRATISRDNGQSTVRLRLNNLRAEDTATYFCAKYAGGYSIDAWGHGTEVIVSS
**SEQ ID NO:4** (ANTI-[TRISP][C9] VL): 
ALTQPSSVSANPGETVKITCSGSSGSYGWYQQKSPGSGPVTVIYYNDKRPSDIPSRFSGSASGST
ATLTITGVQAEDEAVYFCGGYDSSTYVGIFGAGTTLTVL
**SEQ ID NO:5** (ANTI-[TRISP][18H1] VH): 
QVQLKESGPGLVQPSQTLSLTCTVSGLSLTSNSVGWIRQPPGKGLEWMGIIWSNGGTDYNSAIKS
RLSISRDTSKSQVFLKMHSLQTEDTAMYFCARGPYYGSYRLGYFDYWGQGVMVTVSS
**SEQ ID NO:6** (ANTI-[TRISP][18H1] VL):

DVVMTQSPSSLAVSAGETVTLNCQSSQSLLYSGTQNNYLAWYQQKPGQSPKLLISWASTRQSGV
PLRFIGSGSGTDFTLTITSVQAEDLAIYYCQQFYDTPLTFGSGTKLEIK

Die vorliegende Erfindung wird ferner durch die nicht-einschränkenden Beispiele illustriert, denen weitere Merkmale, Ausführungsformen, Aspekte und Vorteile der vorliegenden Erfindung entnommen werden können.

### Beispiel 1: Material und Methoden

| Material | | | |
|---|---|---|---|
| **Material** | **Name** | **Produktnummer** | **Hersteller** |
| **Geräte** | Automated Chemiluminescent Analyzer SuperFlex | | PerkinElmer |
| | Biometra WT 15 042-400 shaker | | Biometra |
| | Centro XS³ LB 960 Mikroplatten Luminometer | | Berthold Technologies |
| | GM 200 Zubehör: Mahlbehälter rostfreier Stahl | 03 045 0050 | Retsch |
| | GM 200 Zubehör: Messer Wellenschliff | 02 446 0057 | Retsch |
| | HydroFlex^{™} Microplate Washer, Magnetic Beads | | Tecan |
| | Inkubator WTB | | BINDER |
| | Messermühle GRINDOMIX GM 200 | 20 253 0001 | Retsch |
| | MTS 2/4 digital microtiter shaker | 3208001 | IKA |
| | Novex Mini Cell | | Invitrogen |
| | Photometer: Sunrise^{™} | | Tecan |
| | Power Pac HV | | Bio-Rad |
| | Tischzentrifuge: Biofuge Fresco | | Heraeus |
| | Washer Columbus | | Tecan |
| | XCell SureLock^{™} Mini-Cell Electrophoresis System | | ThermoFisher Scientific |
| | Zentrifuge Avanti^{®} J-E (Rotor: JA-10) | | Beckman Coulter |
| | Kipp-/Rollenmischer | | IKA |
| **Verbrauchsmaterialien** | iD PAGE Gel, 4-20% | ID-PA4201-015 | Eurogentec |
| | Dynabeads^{™} M-280 Tosylactivated | 14203 | ThermoFisher Scientific |
| | Mikrotiterplatte LockWell Maxisorb | 446475-EUR | ThermoFisher Scientific |
| | Nitrocellulose Membran | 11306 | Sartorius |
| | Nunc^{™} F96 MicroWell^{™} Polystyrolplatte, weiß | 136101 | ThermoFisher Scientific |
| | Reagenz-Kartuschen (SuperFlex) | | PerkinElmer |
| | Schutzkappen für Magnetstab (SuoerFlex) | | PerkinElmer |
| **Medien, Lösungen und Puffer** | Ammoniumsulfatlösung (3 M) | | Sigma-Aldrich |
| | Antikörper-Verdünnungspuffer | | EUROIMMUN |
| | Blockierungspuffer ELISA | | EUROIMMUN |
| | Chromogen/Substratlösung ELISA TMB/H2O2 | ZE 1200-0112 T | EUROIMMUN |
| | Enzymkonjugat Alkalische-Phosphatase-markiertes Anti-Human-IgG (Ziege) | AE 142 1030 | EUROIMMUN |
| | Extravidin/Acridinium-Reagenz | | EUROIMMUN |
| | Beads-Beschichtungspuffer | | EUROIMMUN |
| | PBS | ZF 1100-1000 T | EUROIMMUN |
| | PBS + 0,05% Tween-20 (PBST) | ZF 1110-0102 T | EUROIMMUN |
| | StabilCoat^{®} Plus | SC01 | Surmodics |
| | Stopplösung ELISA (0,5 M Schwefelsäure) | ZE 1210-0112 T | EUROIMMUN |
| | Substratlösung Blot Nitroblautetrazoliumchlorid/5-Brom-4-chlor-3-indolylphosphat (NBT/BCIP) | ZW 1020-0130 T | EUROIMMUN |
| | Trigger A für CLIA | | EUROIMMUN |
| | Trigger B für CLIA | | EUROIMMUN |
| | Tris-Puffer (1 M) | #1218 | Gerbu Biotechnik |
| | Waschpuffer Blot | ZW 1100-1005 T | EUROIMMUN |
| | Waschpuffer ELISA | ZE 11 20-1000 T | EUROIMMUN |
| | Waschpuffer Plus Blot | ZW 1110-1005 | EUROIMMUN |
| **Ready-to-use Lösungen und Kits** | Color Prestained Protein Standard, Broad Range (11-245 kDa) | P7712S | New England BioLabs |
| | NuPAGE^{®} LDS Sample Buffer (4X) | | ThermoFisher Scientific |
| | NuPAGE^{™} MOPS SDS Running Buffer (20X) | NP0001 | ThermoFisher Scientific |
| **Antigene** | *Ascaris suum* | | Prof. Dr. Christina Strube, Tierärztliche Hochschule Hannover, DE |
| | *Salmonella typhimurium* | | Prof. Dr. Michael Hensel Mikrobiologie Universität Osnabrück |
| | *Salmonella* Cholerasuis Stamm A | | |
| | *Salmonella* Cholerasuis Stamm B | | |
| | *Salmonella* Typhisuis | | |
| | *Strongyloides ratti* | | EUROIMMUN |
| | *Toxocara cati* | | EUROIMMUN |
| | *Toxoplasma gondii* | BA110VS | Virion/Serion |
| | *Trichinella spiralis* E/S-Antigen | | EUROIMMUN |
| | *Trichinella spiralis* Lysat | | EUROIMMUN |
| | *Trichuris suis* | | Prof. Dr. Eva Liebau Molecular Physiology Universität Münster |
| | *Trypanosoma cruzi* | | Virion/Serion |
| **Antikörper** | Anti-[TRISP][B7] | | EUROIMMUN |
| | Anti-[TRISP][C9] | | EUROIMMUN |
| | Anti-[TRISP][18H1] | | EUROIMMUN/ Prof. Dr. Judith Appleton, Cornell University, Ithaca, USA |
| **Sonstige Materialien** | EZ-Link^{™} NHS-PEG12-Biotin | 21312 | ThermoFisher Scientific |
| | IIFT *Trichinella spiralis* "Eingekapselte Larve" | | EUROIMMUN |
| | IIFT *Trichinella spiralis* "Muskellarve" | | EUROIMMUN |
| | Schweinefleischproben, versetzt mit einer definierten Anzahl an Trichinella-Muskellarven | | Bundesinstitut für Risikobewertung (BfR) |
| | Schweinefleisch Zwerchfellpfeiler | | Fleischerei Prösch, Krummesse |
| | Spectra^{™} Multicolor Broad Range Protein Ladder | | ThermoFisher Scientific |
| | Streptavidin-PolyHRP80, stock 1 mg/ml solution | #SP80C | SDT (Stereospecific Detection Technologies) |
| | Trichinöses Schweinefleisch, infiziert mit 20 bis 50 Larven pro Gramm | | Bundesinstitut für Risikobewertung (BfR) |
| | Zeba^{™} Spin Desalting Columns, 40K MWCO, 10 mL | 87772 | ThermoFisher Scientific |

### Methoden

### Verfahren zur Gewinnung von Gewebeextraktproben

In der vorliegenden Nachweismethode soll das Fleisch nicht enzymatisch verdaut, sondern mechanisch zerkleinert werden. Dabei soll eine Partikelgröße des Fleisches von < 200 µm erreicht werden. Die Larven sind mit der Kollagen-Kapsel ca. 200 - 600 µm lang und 200 - 300 µm breit, sodass man bei einer gewünschten Partikelgröße von < 200 µm davon ausgehen kann, dass die eingekapselten *Trichinella-Larven* zumindest einmal von dem Schneidemesser des Zerkleinerungsgerätes erfasst werden müssen. In der Kapsel befinden sich zahlreiche E/S-Proteine, die bei der Zerkleinerung freigesetzt werden und sich dann frei im Probenmaterial befinden (Figur 1). Die somatischen Proteine können durch die Zerkleinerung der Larve zusätzlich detektiert werden.

Im Anschluss an die Zerkleinerung erfolgt ein Antigen-Nachweis in Form eines *Antigen-Capture ELISA* oder eines manuell inkubierten bzw. automatisierten Chemilumineszenz Immunassays.

### Probenvorbereitung

Auf dem Schlachthof ist die Entnahme von ca. 5 g Fleisch pro Tier üblich. Die Probe wird standardmäßig aus dem muskulösen Teil des Zwerchfells eines bereits getöteten Tieres entnommen. Für die Trichinenuntersuchung werden pro Schwein 1 g Probenmaterial verwendet. Für andere Körperteile wie Zunge oder Zwischenrippenmuskulatur kann die Probenmenge abweichen. Es werden in der Regel 100 Schweineproben gepoolt, sodass eine Probenmenge von 100 x 1 g entsteht. Die Probe wird auf 4 °C heruntergekühlt.

### Zerkleinerung der Fleischproben

Das Probenmaterial (100 x 1 g Fleisch) wurde in den auf 4°C vorgekühlten Mahlbecher eines Zerkleinerungsgerätes gegeben. Dafür wurde die Messermühle Grindomix GM 200 von Retsch verwendet. Das Zerkleinerungsprinzip beruht auf dem Zerschneiden der Probe. Die Messermühle kann mit einem Wellenschliffmesser ausgestattet werden, sodass auch faserige Materialien wie Muskelgewebe fein zerkleinert werden können. Zu der Probe wurden 200 ml 4°C kaltes PBS gegeben. Bei 10.000 rpm und damit maximaler Leistung wurde das Probenmaterial wie angegeben, z.B. 9 Min. lang, zerkleinert.

Um zu überprüfen, ob die Zerkleinerung des Fleisches erfolgreich war, wurde eine Partikelgrößenmessung mit dem CamsizerOXT von der Firma Retsch und dem LA-960 von der Firma HORIBA durchgeführt. Der LA-960 basiert auf dem Funktionsprinzip der statischen Laserstreuung, wohingegen der CamsizerOXT auf einer dynamischen Bildanalyse beruht. Es wurden jeweils drei Fleischproben nach 6 Min. bzw. 9 Min. entnommen und direkt im Anschluss vermessen. Vermessen wurden im Durchschnitt 60 Mio. Partikel. Die Partikelgröße wurde in x_{c min} gemessen und nach DIN 66141 folgendermaßen definiert: Kürzester Partikeldurchmesser der Messungen der maximalen Durchmesser innerhalb einer Partikelprojektion (englisch: *particle diameter which is the shortest chord of the measured set of maximum chords of a particle projection)* [8].

### Zentrifugation

Nach der Zerkleinerung wurden dem Mahlbecher 1 ml bis 15 ml Probe mit einer Pipette entnommen. Es folgte die Sedimentierung der groben Partikel in der Probe bei einer 10-minütigen Zentrifugation bei 5.000 x g und 4°C. Der Überstand nach der Sedimentation ist das Probenmaterial für den nachfolgenden Antigen-Nachweis und wird als Gewebeextrakt bezeichnet.

### Verfahren zum Nachweis von Trichinella spiralis aus Gewebeextraktproben

### Herstellung des Probenmaterials

*T. spiralis* Lysat
*T. spiralis* Muskellarven (ML) wurden von Justyna Bień vom Witold Stefański Institut PAS in Warschau zur Verfügung gestellt. 120.000 ML wurden 10 Min. zentrifugiert bei 16.000 x g und Raumtemperatur (RT), sodass alle Larven pelletiert waren. Der Überstand wurde verworfen und 1 ml PBS hinzugegeben. Die Larven wurden fünf Einfrier- und Auftau-Zyklen ausgesetzt und anschließend mit einem Hand-Homogenisator zerkleinert. Schließlich wurden die Proben mit Ultraschall behandelt: 20 Zyklen jeweils 5 Sek. bei mittlerer Stärke und zwischen den Behandlungen jeweils 5 Sek. Pause auf Eis. Es folgte eine 20-minütige Zentrifugation bei 16.000 x g und 4°C. Der Überstand stellt das Antigen *T. spiralis* Lysat dar.

### E/S-Antigen

Das E/S-Antigen von *T. spiralis* ML wurde von Justyna Bień vom Witold Stefański Institut PAS in Warschau zur Verfügung gestellt.

### Herstellung der Antikörper

Die Antikörper, die für den *Antigen-Capture ELISA* eingesetzt werden, wurden mittels *Phage Display* Methode hergestellt [9]. Die Sequenzen der variablen Regionen der schweren und leichten Kette (VH und VL) der beiden Antikörper Anti-[TRISP][B7] und Anti-[TRISP][C9] (kurz: AK B7 und AK C9) lauten folgendermaßen:
Anti-[TRISP][B7] VH (SEQ ID NO:1): 
AVTLDESGGGLQTPRGGLSLVCKASGYTFSSHNMAWVRQAPGKGLEFVAGISNTGSFTLYGAAV
KGRATISRDNGQSTVRLQLNNLRAEDTGTYYCAKHAGVGLYSIDAWGHGTEVIVSS
Anti-[TRISP][B7] VL (SEQ ID NO:2): 
ALTQPSSVSANLGGTVKITCSGGTSDYGWYQQKAPGSAPVTLIYDNTNRPSDIPSRFSGSLSGST
NTLTITGVQAEDEAVYFCGSADRTYAGVFGAGTTLTVL
ANTI-[TRISP][C9] VH (SEQ ID NO:3): 
AVTLDESGGGLQTPGGALSLVCKASGFSISSYSMQWVRQAPGKGLEWVAGIYYDGNTWYAPAVK
GRATISRDNGQSTVRLRLNNLRAEDTATYFCAKYAGGYSIDAWGHGTEVIVSS
ANTI-[TRISP][C9] VL (SEQ ID NO:4):

ALTQPSSVSANPGETVKITCSGSSGSYGWYQQKSPGSGPVTVIYYNDKRPSDIPSRFSGSASGST
ATLTITGVQAEDEAVYFCGGYDSSTYVGIFGAGTTLTVL

### Biotinylierung des Antikörpers Anti-[TRISP][B7]

Der AK B7 wurde für den *Antigen-Capture ELISA* mit EZ-Link^{™} NHS-PEG12-Biotin in einem 20-fachen molaren Überschuss für eine Stunde bei Raumtemperatur auf dem Rotationsschüttler inkubiert. Um überschüssiges Biotin zu entfernen, wurde der Antikörper (AK) nach Anleitung des Herstellers über eine Größenausschlusschromatografie-Säule (Zeba^{™} Spin Desalting Columns) aufgereinigt.

### SDS PAGE und Westernblot-Analyse

Für die SDS PAGE wurden jeweils 5 µg *T. spiralis* Lysat bzw. Lysat von *Trypanosoma cruzi, Ascaris suum, Strongyloides ratti, Toxocara cati, Toxoplasma gondii, Salmonella typhimurium, Salmonella cholerasuis* Stamm A, *Salmonella cholerasuis* Stamm B, *Salmonella typhisuis* und *Trichuris suis* auf ein Polyacrylamid Gel geladen und die Elektrophorese wurde 50 Min. bei 175 V in MOPS-Puffer durchgeführt. Der Transfer erfolgte auf eine Nitrocellulose-Membran für 60 Min. bei 400 mA in Transferpuffer. Die Membran wurde für die Blockierung in Waschpuffer Plus für 30 Min. auf dem Wippschüttler inkubiert. Anschließend wurden die AK B7 und C9 in Waschpuffer Plus mit einer Konzentration von 0,4 µg/ml aufgebracht und über Nacht auf dem Wippschüttler inkubiert. Nach dem Waschen mit Waschpuffer wurde die Membran mit dem in Waschpuffer Plus verdünnten Enzymkonjugat "Alkalische-Phosphatase-markiertes Anti-Human-IgG" von EUROIMMUN inkubiert. Schließlich wurde nach einem erneuten Waschschritt die Substratlösung (NBT/BCIP) aufgegeben und inkubiert bis ein deutlicher Farbumschlag zu sehen war.

### Indirekter Immunfluoreszenztest

Um zu überprüfen, welche Strukturen die entwickelten AK binden, wurde ein indirekter Immunfluoreszenztest für *T. spiralis* entwickelt. Die BIOCHIPS waren mit Gefrierschnitten von *T. spiralis* Muskellarven und eingekapselten Larven bestückt. Die Inkubation und die Mikroskopie erfolgten nach der Anleitung des *Anti-Schistosoma-mansoni-*IIFTs von EUROIMMUN (Bestellnr. FI 2300-1005 G). Als Probe wurden die AK B7 und C9 mit einer Konzentration von 2 µg/ml auf die BIOCHIPS gegeben.

### Antigen-Nachweis mittels Antigen-Capture ELISA

Für die Antigen-Detektion wurde ein *Enzyme-linked Immunosorbent Assay* (ELISA) als Nachweisverfahren verwendet. Eine 96-Well Mikrotiterplatte wurde mit 0,25 µg/ml AK C9 in PBS über Nacht bei 4°C beschichtet. Am nächsten Tag wurde die Mikrotiterplatte mit PBST (PBS + 0,05% Tween-20) einmal gewaschen, mit Blockierungspuffer für 2 Stunden blockiert und anschließend 2 Stunden lang getrocknet.

Als Nachweis für die an AK C9 gebundenen Antigene wurde der AK B7 mit einer Konzentration von 0,05 µg/ml und Streptavidin-PolyHRP80 mit einer Konzentration von 0,1 µg/ml zusammen in AK-Verdünnungspuffer angesetzt und über Nacht inkubiert.

### Inkubation der Proben

Die Inkubation erfolgte wie auf dem in Figur 2 dargestellten Schema. Als Proben wurden das E/S-Antigen von *T. spiralis* in verschiedenen Konzentrationen (0-50 ng/ml) und Gewebeextraktproben in einem Volumen von 100 µl auf die Mikrotiterplatte aufgebracht. Die Inkubation erfolgte für eine Stunde bei Raumtemperatur auf einem Rotationsschüttler. Die "positive" Gewebeextraktprobe wurde hergestellt, indem vor der Zerkleinerung trichinöses Schweinefleisch zu negativem Zwerchfellpfeilergewebe gegeben wurde, sodass sich ca. 100 eingekapselte *Trichinella-Larven* in der Probe befanden.

Nach sechsmaligem Waschen mit Waschpuffer wurde das Konjugat mit einem Volumen von 100 µl ebenfalls für eine Stunde bei Raumtemperatur auf einem Rotationsschüttler inkubiert. Anschließend wurde erneut sechsmal gewaschen und das Substrat aufgebracht. Nach 15 Min. wurde die Reaktion mit Stopplösung beendet und die Optische Dichte (O.D.) der Proben mit einem Photometer bei einer Wellenlänge von 450 nm bestimmt.

### Beispiel 2: Ergebnisse

### Zerkleinerung der Fleischproben

Direkt im Anschluss an die Zerkleinerung des Schweinefleisches mit der Messermühle GM200 wurde eine Partikelgrößenbestimmung mit dem Camsize^{®}XT und dem HORIBA LA-960 durchgeführt. Die Probenahme erfolgte nach 6 bzw. 9 Min. Die Ergebnisse der Massenverteilung sind in Figur 3 zu sehen, wo die Partikelgröße [µm] in einem Diagramm gegen Q3 [%] aufgetragen ist. Q3 ist der Anteil an Partikeln in Prozent, die kleiner sind als x in Bezug auf das gesamte Volumen. Da die beiden Partikelgrößenmessgeräte auf unterschiedlichen Messverfahren beruhen, weichen die Ergebnisse voneinander ab.

Die Ergebnisse der Messung mit dem HORIBA LA-960 zeigen, dass 95% der Partikel < 26 µm und alle Partikel < 100 µm sind. Mit der Camsize^{®}XT-Messung sind 95% der Partikel < 300 µm. Nur 70% der Fleischpartikel sind < 100 µm. In Figur 4 ist exemplarisch dargestellt, welche Form die Fleisch-Partikel haben. Durch die Muskelfasern und Myofibrillen ist die Struktur sehr faserig, was das Breiten- zum Längenverhältnis deutlich herabsenkt.

In jedem Fall würde die Kollagenkapsel einer *Trichinella*-Larve, wenn vorhanden, statistisch mindestens einmal durch das Messer angeschnitten werden, sodass E/S-Proteine freigesetzt werden können. Diese freigesetzten Proteine sind im nächsten Schritt mit einem *Antigen-Capture ELISA* detektierbar.

### Herstellung des Probenmaterials und der Antikörper

Um zu überprüfen, ob die Antikörper Anti-[TRISP][B7] und Anti-[TRISP][C9] für einen *T. spiralis* Antigen-Nachweis geeignet sind, wurden als Funktionsprüfung ein indirekter Immunfluoreszenztest (IIFT) und ein Westernblot durchgeführt. Die Ergebnisse sind in Figur 5 dargestellt.

Bei Verwendung von AK B7 fluoresziert die angeschnittene Kapsel am stärksten (Figur 5 A). Die Larve im Inneren und einzelne Proteine auf der Kapsel-Oberfläche fluoreszieren ebenfalls. Der Gefrierschnitt der Muskellarve zeigt eine deutliche Reaktion an der äußeren Membran (Figur 5 B).

Im Querschnitt der eingekapselten Larve fluoresziert die gesamte Larve am stärksten bei der Inkubation mit dem AK C9 (Figur 5 C). Die einzelnen Proteine auf der Kapsel-Oberfläche und die angeschnittene Kapsel fluoreszieren schwach. Bei der Muskellarve zeigt nicht nur die äußere Membran eine deutliche Reaktion, sondern auch das Innere der Larve (Figur 5 D).

Im Westernblot sind ebenfalls deutliche Reaktionen für die AK B7 und C9 zu sehen (Figur 5 E). Die beiden AK zeigen ein identisches Bandenmuster. Starke Banden treten bei ca. 48, 50, 52, 60, 65 und 110 kDa auf. Das Epitop, das von den AK gebunden wird, scheint bei mehreren Proteinen bzw. Glykoproteinen vorzukommen, was sich positiv auf die Detektion von sehr gering konzentrierten *Trichinella*-Antigenen im Probenmaterial auswirken kann.

Um zu überprüfen, ob die AK ausschließlich spezifische Strukturen von *T. spiralis* binden und keine antigenen Strukturen von anderen Parasiten und/oder Bakterien, wurde ein Westerblot durchgeführt. Dabei wurden Lysate von Pathogenen aufgetragen, die bei Schweinen vorkommen. Das Ergebnis ist in Figur 6 zu sehen. Die getesteten Lysate besitzen keine Strukturen, die von AK B7 oder AK C9 gebunden werden. Eine falsch positive Reaktion kann im späteren *Antigen-Capture ELISA* für diese Pathogene mit hoher Wahrscheinlichkeit ausgeschlossen werden.

### Antigen-Capture ELISA

Für die Funktionsprüfung des entwickelten *T. spiralis* ELISA wurden verschiedene Konzentrationen an E/S-Antigen im Test eingesetzt (Figur 7 A). Die Detektionsgrenze liegt mit einem *Cut-off* von 0,3 O.D. bei ca. 6 ng/ml.

In der Figur 7 B wurden Ergebnisse von Gewebeextraktproben dargestellt, die nach dem unter hierin beschriebenen Verfahren hergestellt wurden. Getestet wurden sowohl eine positive Probe, bei der sich ca. 100 eingekapselte Larven im Schweinefleisch befanden als auch eine negative Probe. Der *Antigen-Capture ELISA* zeigt einen deutlichen Unterschied von fast 2 O.D zwischen der positiven und der negativen Gewebeextraktprobe.

Die *T. spiralis* Antigen-Konzentration in der Gewebeextraktprobe entspricht ca. 30 ng/ml E/S-Antigen. 100 zerkleinerte eingekapselte Larven setzen folglich in etwa diese Menge an Proteinen in den Fleischsaft frei, die durch den *Antigen-Capture ELISA* detektierbar sind.

### Antigen-Capture ELISA mit unterschiedlich zerkleinertem Probenmaterial

In Figur 8 sind die Ergebnisse der Zerkleinerung nach 3, 6 und 9 Min. von positivem und negativem Gewebeextrakt dargestellt. Es ist zu sehen, dass der negative Gewebeextrakt ohne Larven im Probenmaterial keine signifikante Reaktion zeigt, da die O.D. im ELISA unter dem *Cut-Off* von 0,3 liegt. Der Gewebeextrakt, der aus Fleisch hergestellt wurde und in dem sich ca. 100 eingekapselte *T. spiralis* Larven befanden, zeigt hingegen eine deutliche Reaktion mit > 1,5 O.D. Auffällig sind die Auswirkungen durch den Umfang der Zerkleinerung, da die Reaktion mit zunehmender Zerkleinerungszeit ansteigt. Die O.D. bei der 9-minütigen Zerkleinerung ist um 0,5 höher als bei der 3-minütigen Zerkleinerung.

Die Reaktion im ELISA ist bei dem Probenmaterial, das 3 Min. zerkleinert wurde, geringer als nach einer Zerkleinerung für 9 Min. Eine längere Zerkleinerung des Probenmaterials führt folglich zu einer höheren Menge an freigesetztem *T. spiralis* Antigen. Durch eine verlängerte Zerkleinerung werden wahrscheinlich alle eingekapselten *Trichinella*-Larven zumindest einmal und meistens mehrfach von dem Schneidemesser des Zerkleinerungsgerätes erfasst, sodass im Vergleich zu einer verkürzten Zerkleinerung mehr detektierbare Antigene für den ELISA im Probenmaterial verfügbar sind.

### Beispiel 3: Antigen-Nachweis mittels eines manuell inkubierten und automatisierten Chemilumineszenz Immunassays

Für den manuell inkubierten und Bead-basierten Chemilumineszenz Immunassay (CLIA) wurden die Fängerantikörper nicht an einer Mikrotiterplatte, sondern an magnetische Beads gekoppelt. Dafür wurden Tosyl-aktivierte Dynabeads^{™} verwendet. Die hydrophobe Polyurethanoberfläche war dabei mit Tosyl-Gruppen aktiviert, wodurch die Antikörper kovalent mit den Beads verbunden werden konnten. 4 mg der Beads wurden mit 1 ml eines 1 M Tris-Puffers equilibriert. Zu den equilibrierten Beads wurden 30 mM Tris, 0,4 M Ammoniumsulfat und 28 µg Anti-[TRISP][18H1]-Antikörper (kurz: AK 18H1) gegeben. Die Inkubation erfolgte über Nacht bei 37°C auf einem Rollenmischer. Am nächsten Tag wurden die Beads dreimal mit je 1 ml StabilCoat^{®} Plus gewaschen und dann über Nacht bei 37°C auf dem Rollenmischer inkubiert, um noch reaktive funktionelle Gruppen zu blockieren. Nach der Blockierung wurden die Beads in frischem StabilCoat^{®} Plus aufgenommen und mit einer Konzentration von 1 mg/ml bei 4°C bis zur Verwendung gelagert.

Die mit den 18H1-AK beschichteten Beads wurden für den CLIA zu je 10 µg in ein Mikrotiterplatten-Well (NuncTM Polystyrolplatte) pipettiert. 100 µI der unverdünnten Gewebeextraktprobe wurden dazugegeben und für 30 min auf einem Rotationsschüttler inkubiert. Nach dreimaligem automatisiertem Waschen mit dem HydroFlex^{™} Microplate Washer wurden 100 µl des biotinylierten B7-AKs mit einer Konzentration von 0,1 µg/ml für 30 min auf dem Rotationsschüttler inkubiert. Es folgte dreimaliges automatisiertes Waschen. Für die Chemilumineszenz-Reaktion wurde das Extravidin/Acridinium-Reagenz mit einer Konzentration von 80 ng/ml und einem Volumen von 100 µl zu den Beads gegeben und für 15 min inkubiert. Nach dreimaligem Waschen erfolgte die Messung mit dem Centro XS³ Mikroplatten Luminometer. Das Luminometer gab automatisch zu jedem Ansatz 100 µl der Trigger A und B hinzu, um die Chemilumineszenz-Reaktion zu starten. Die Licht-Emission wurde bei 425 nm Wellenlänge 1 Sek. lang gemessen und in relativen Lichteinheiten (Relative Light Units, RLU) angegeben. Das Inkubationsschema des manuell inkubierten CLIA ist in Fig. 9 zu sehen.

Der automatisierte und Bead-basierte Chemilumineszenz Immunassay beruht auf dem Aufbau des manuell inkubierten CLIA. Der Unterschied ist, dass der Test mit Hilfe eines automatisierten Chemilumineszenz Analysegerätes (Automated Chemiluminescent Analyzer SuperFlex, PerkinElmer) abgearbeitet wird. Die Beads werden dabei von einem magnetischen Stab nacheinander in die verschiedenen Reagenzien getaucht. Der Magnetstab ist in der Lage ein elektrisches Feld zu erzeugen und kann die Beads so aufnehmen, abgeben und die Reagenzien in einem vordefinierten Intervall durchmischen.

Für den automatisierten CLIA wurden die Gewebeextraktproben in das Probenfach des Analysegerätes geladen. Die Reagenz-Kartuschen wurden befüllt wie in Fig. 10 dargestellt. Pro Reaktion wurden 10 µg von den mit 18H1-AK beschichteten Beads eingesetzt. Diese wurden mit 100 µl Gewebeextraktprobe und 100 µl biotinylierten Anti-TRISP[B7]-AK mit einer Konzentration von 0,25 µg/ml für 30 Min. inkubiert. Die Beads wurden nach der Inkubation mit dem Magnetstab aufgenommen und 3x in Waschpuffer (V = 400 µl) gewaschen. Anschließend erfolgte eine 10-minütige Inkubation mit 100 µl 0,1 µg/ml Extravidin/Acridinium-Reagenz. Nach dreimaligem Waschen wurden die Beads in 100 µl Trigger A gegeben und Trigger B (V = 100 µl) wurde vom Analysegerät hinzu pipettiert, um die Chemilumineszenz-Reaktion zu starten. Die Licht-Emission wurde bei 425 nm Wellenlänge 1 Sek. lang von dem in das Analysegerät eingebauten Luminometer gemessen und in relativen Lichteinheiten (Relative Light Units, RLU) angegeben.

### Ergebnisse des manuell inkubierten Chemilumineszenz Immunassays (CLIA)

Es wurden verschiedene Konzentrationen an *T. spiralis* Lysat-Proben mit dem manuell inkubierten CLIA inkubiert. Die Nachweisgrenze lag bei 1 ng/ml *T. spiralis* Lysat.

### Ergebnisse des automatisierten Chemilumineszenz Immunassays (CLIA)

Es wurden ebenfalls verschiedene Konzentrationen an Lysat-Proben von *T. spiralis* mit dem automatisierten Chemilumineszenz Immunassay inkubiert. Die Nachweisgrenze lag bei 10 pg/ml *T*. *spiralis* Lysat. Neben den mit *Trichinella* infizierten Fleischproben wurden auch Fleischproben zerkleinert, die mit einer definierten Menge an *Trichinella*-Muskellarven versetzt waren. Sie waren mit 3 bis 30 Larven bestückt. Die Nachweisgrenze des automatisierten CLIA lag für die definierte Menge an Muskellarven bei 3 Larven pro 100 Gramm Fleisch.

In Fig. 11 wird die Korrelation zwischen der Partikelgröße und den Ergebnissen des CLIA von zerkleinertem und *Trichinella*-positivem Gewebeextrakt dargestellt. Es ist zu sehen, dass die Partikelgröße des Fleisches mit fortschreitender Zerkleinerungszeit abnimmt, die Reaktion des CLIA dagegen zunimmt. Mit fortschreitender Zerkleinerung des Fleisches, und damit auch der Larven, werden vermehrt *Trichinella-*Antigene freigesetzt. Diese können dann durch den CLIA detektiert werden. Die relativen Lichteinheiten (RLU) nehmen auch mit steigernder Larvenanzahl in der Fleischprobe zu. Der *Trichinella-*positive Gewebeextrakt, der aus Fleischproben versetzt mit 30 Larven entstand, zeigt nach 6 Min. Zerkleinerung eine mehr als dreimal Mal so hohe Reaktion (150.000 RLU) wie der Gewebeextrakt von 6 Larven (50.000 RLU).

Die Erfindung wird hierin generisch und allgemein beschrieben. Jede der engeren Arten und Untergruppen, die unter die generische Offenbarung fallen, bilden auch einen Teil der Erfindung. Dies schließt die allgemeine Beschreibung der Erfindung mit einer Vorbehalts- oder Negativbeschränkung ein, die jeden Gegenstand von einer (Unter-)Gruppe entfernt, unabhängig davon, ob der ausgeschnittene Gegenstand hier spezifisch zitiert ist oder nicht. Andere Ausführungsformen sind in den folgenden Ansprüchen enthalten.

Ein Fachmann wird leicht erkennen, dass die vorliegende Erfindung gut dazu geeignet ist, die Aufgaben auszuführen und die erwähnten Vorteile sowie die damit verbundenen Ziele zu erreichen. Ferner ist es für den Fachmann ohne weiteres ersichtlich, dass verschiedene Substitutionen und Modifikationen an der hier offenbarten Erfindung vorgenommen werden können, ohne von der Erfindung abzuweichen. Die hierin beschriebenen Verfahren sind repräsentativ für bevorzugte Ausführungsformen, die beispielhaft sind und nicht als Beschränkungen des Umfangs der Erfindung gedacht sind. Änderungen darin und andere Verwendungen werden dem Fachmann einfallen, die im Umfang der Erfindung eingeschlossen sind und durch den Umfang der Ansprüche definiert sind. Die Auflistung oder Diskussion eines zuvor veröffentlichten Dokuments in dieser Beschreibung sollte nicht notwendigerweise als Bestätigung dafür verstanden werden, dass das Dokument zum Stand der Technik gehört oder allgemein bekannt ist.

Die hierin veranschaulichend beschriebene Erfindung kann in geeigneter Weise in Abwesenheit von irgendeinem Element oder Beschränkungen, die hier nicht speziell offenbart sind, ausgeführt werden. So werden beispielsweise die Ausdrücke "umfassend", "einschließlich", "enthaltend" usw. umfassend und ohne Einschränkung gelesen. Das Wort "umfassen" oder Variationen, wie "umfasst" oder "umfassend", werden dementsprechend als implizit verstanden, d.h. beispielsweise, dass angegebene Zahlen einbezogen, aber nicht ausgeschlossen werden. Zusätzlich wurden die hierin verwendeten Begriffe und Ausdrücke als Ausdrücke der Beschreibung und nicht der Begrenzung verwendet, und es gibt keine Absicht solche Begriffe und Ausdrücke, um jegliche Äquivalente der gezeigten und beschriebenen Merkmale oder Teile davon zu begrenzen. D.h. dass verschiedene Modifikationen innerhalb des Schutzumfangs der beanspruchten Erfindung möglich sind. Somit sollte verstanden werden, dass, obwohl die vorliegende Erfindung durch beispielhafte Ausführungsformen und optionale Merkmale spezifisch offenbart worden ist, Modifikationen und Variationen der Erfindungen, die hierin offenbart sind, von Fachleuten auf dem Feld verwendet werden können, und dass solche Modifikationen und Variationen als innerhalb des Schutzumfangs dieser Erfindung anzusehen sind.

### Abkürzungen

| **Abkürzung** | **Englisch** | **Deutsch** |
|---|---|---|
| µg | | Mikrogramm |
| µl | | Mikroliter |
| AG | | Antigen |
| AK | | Antikörper |
| CLIA | Chemiluminescent Immunoassay | Chemilumineszenz Immunassay |
| E/S | Excretory/Secretory | Exkretorisch/Sekretorisch |
| ELISA | Enzyme-linked Immunosorbent Assay | |
| EU | | Europäische Union |
| 9 | | Gramm |
| IIFT | | Indirekter Immunfluoreszenztest |
| I | | Liter |
| mg | | Milligramm |
| Min. | | Minute |
| ml | | Milliliter |
| ML | | Muskellarven |
| NBL | New Born Larvae | Neugeborene Larven |
| NBT/BCIP | | Nitroblautetrazoliumchlorid/5-Brom-4-chlor-3-indolylphosphat |
| ng | | Nanogramm |
| nm | | Nanometer |
| O.D. | | Optische Dichte |
| rpm | rounds per minute | Umdrehungen pro Minute |
| RT | | Raumtemperatur |
| Sek. | | Sekunde |
| SDS PAGE | Sodium Dodecyl Sulfate Polyacrylamide Gel Electrophoresis | Natriumdodecylsulfat-Polyacrylamidgelelektrophorese |

### Literatur

1. Dupouy J, Murrell KD: FAO/WHO/OIE guidelines for the surveillance, management, prevention and control of trichinellosis; 2007.
2. Mitreva M, Jasmer DP: WormBook: Biology and genome of Trichinella spiralis; 2006.
3. Zarlenga DS, La Rosa G, Pozio E, Rosenthal B: Identification and classification within the genus Trichinella, with special emphasis on non-encapsulated species. Vet Parasitol 2004, 125:75-78.
4. Liu M, Boireau P: Trichinellosis in China: epidemiology and control. Trends Parasitol 2002, 18(12):553-556.
5. BfR: Trichinellose - Erkennung, Behandlung und Verhütung. Information BfR 2007.
6. Kapel CM: Changes in the EU legislation on Trichinella inspection--new challenges in the epidemiology. Vet Parasitol 2005, 132(1-2):189-194.
7. Nöckler K, Serrano FJ, Boireau P, Kapel CM, Pozio E: Experimental studies in pigs on Trichinella detection in different diagnostic matrices. Vet Parasitol 2005, 132(1-2):85-90.
8. RKI: Ringversuch zum Nachweis von Trichinellen in Fleisch. In.: Robert Koch Institut; 2016.
9. Report on the validation of the Trichin-L antigen test kit of the Bio-Rad company In.: European Union Reference Laboratory for Parasites; 2010.
10. Retsch GmbH: CAMSIZER® Characteristics - Basis of definition DIN 66141. 2009.
11. Appleton J A, Schain LR, and McGregor DD. 1988. Rapid expulsion of Trichinella spiralis in suckling rats: mediation by monoclonal antibodies. Immunology 65: 487-492.
12. Altschul SF, Gish, W, Miller W, Myers EW & Lipman DJ. (1990) Basic local alignment search tool. J. Mol. Biol. 215:403- 410
13. Altschul SF, Madden TL, Schaffer AA, Zhang J, Zhang H, Miller W, and Lipman DJ. (1997): Gapped BLAST and PSI-BLAST: a new genera- tion of protein database search programs; Nucleic Acids Res., 25, S.3389-3402
14. Chenna et al. (2003): Multiple sequence alignment with the Clustal series of programs. Nucleic Acid Research 31, 3497-3500
15. Notredame et al. (2000): T-Coffee: A novel method for multiple sequence alignments. J. Mol. Biol. 302, 205-217

## Patentansprüche

1. Verfahren zur Detektion von *Trichinella* in einer Gewebeextraktprobe, wobei in der Gewebeextraktprobe mindestens 90% der Partikel einen Durchmesser von 200 µm oder weniger (D90 ≤ 200 µm) haben und wobei bei der Herstellung der Gewebeextraktprobe keine enzymatische und/oder chemische Spaltung des Gewebes erfolgt.

2. Das Verfahren gemäß Anspruch 1, wobei die Gewebeextraktprobe eine Säugetierprobe, bevorzugt eine Probe von einem Schwein, ist.

3. Das Verfahren gemäß einem der Ansprüche 1 oder 2, wobei die Gewebeextraktprobe aus Muskulatur stammt.

4. Das Verfahren gemäß einem der Ansprüche 1 bis 3, wobei bei der Herstellung der Gewebeextraktprobe eine Temperatur von 45 °C, bevorzugt 40 °C, nicht überschritten wird.

5. Das Verfahren gemäß einem der Ansprüche 1 bis 4, wobei das Verfahren
(a) keinen Mikroskopierschritt umfasst;
(b) im Rahmen der Fleischbeschau verwendet wird; und/oder
(c) eine Nachweisgrenze von ≦ 7 ng Antigen pro ml Gewebeextrakt besitzt.

6. Das Verfahren gemäß einem der Ansprüche 1 bis 5, wobei das Verfahren durch einen Immunassay, bevorzugt durch einen ELISA, Lateral-Flow Assay, Lineblot-Assay, Western Blot-Assay, bead-basierten Assay, vertikalen Filtrationsassay oder 3D-Immunofiltrationsassay durchgeführt wird.

7. Das Verfahren gemäß einem der Ansprüche 1 bis 6, wobei *Trichinella Trichinella spiralis* ist.

## Claims

1. A method of detecting *Trichinella* in a tissue extract sample, wherein at least 90% of the particles in the tissue extract sample have a diameter of 200 µm or less (D90 ≤ 200 µm) and wherein in the preparation of the tissue extract sample no enzymatic and/or chemical cleavage of the tissue takes place.

2. The method according to claim 1, wherein the tissue extract sample is a mammalian sample, preferably a sample from a pig.

3. The method according to one of claims 1 or 2, wherein the tissue extract sample is from musculature.

4. The method according to one of claims 1 to 3, wherein in the preparation of the tissue extract sample a temperature of 45 °C, preferably 40 °C, is not exceeded.

5. The method according to one of claims 1 to 4, wherein the method
(a) does not comprise a microscopy step;
(b) is used in meat inspection; and/or
(c) has a detection limit of ≤ 7 ng antigen per ml of tissue extract.

6. The method according to one of claims 1 to 5, wherein the method is performed by means of an immunoassay, preferably by means of an ELISA, lateral flow assay, line blot assay, Western blot assay, bead-based assay, vertical filtration assay or 3D immunofiltration assay.

7. The method according to one of claims 1 to 6, wherein *Trichinella* is *Trichinella spiralis.*

## Revendications

1. Procédé de détection de *Trichinella* dans un échantillon d'extrait tissulaire, au moins 90 % des particules de l'échantillon d'extrait tissulaire ayant un diamètre de 200 µm ou moins (D90 ≤ 200 µm), et aucune dissociation enzymatique et/ou chimique du tissu ne se produisant lors de la fabrication de l'échantillon d'extrait tissulaire.

2. Procédé selon la revendication 1, dans lequel l'échantillon d'extrait tissulaire est un échantillon de mammifère, de préférence un échantillon de porc.

3. Procédé selon l'une des revendications 1 ou 2, dans lequel l'échantillon d'extrait tissulaire provient de muscles.

4. Procédé selon l'une des revendications 1 à 3, dans lequel, lors de la fabrication de l'échantillon d'extrait tissulaire, on ne dépasse pas une température de 45 °C, de préférence de 40 °C.

5. Procédé selon l'une des revendications 1 à 4, le procédé
(a) ne comprenant aucune étape d'observation au microscope ;
(b) étant utilisé dans le cadre de l'inspection des viandes ; et/ou
(c) présentant une limite de détection ≤ 7 ng d'antigène par ml d'extrait tissulaire.

6. Procédé selon l'une des revendications 1 à 5, le procédé étant mis en oeuvre par un immuno-essai, de préférence un ELISA, un essai d'écoulement latéral, un essai Lineblot, un essai par transfert de Western, un essai à base de perles, un essai de filtration verticale ou un essai d'immuno-filtration 3D.

7. Procédé selon l'une des revendications 1 à 6, dans lequel *Trichinella* est *Trichinella spiralis.*
